# EUROPEAN PATENT SPECIFICATION

(11) **EP 2 209 392 B1**
(45) Date of publication and mention of the grant of the patent: **01.01.2014**
(21) Application number: 08800093.0
(22) Date of filing: 03.10.2008
(51) Int. Cl.: A23L 3/3562, A61K 8/60, C09K 15/00, A23L 1/30, A61K 36/889, C09K 15/34

(54) **NATURAL PRESERVATIVES AND ANTIMICROBIAL AGENTS**
NATÜRLICHE KONSERVIERUNGSMITTEL UND ANTIMIKROBIELLE WIRKSTOFFE
AGENTS CONSERVATEURS ET ANTIMICROBIENS NATURELS

(30) Priority: 05.10.2007 AU 2007905459
(43) Date of publication of application: 28.07.2010
(73) Proprietor: Horizon Science Pty Ltd, Braeside VIC 3195 (AU)
(72) Inventor: KANNAR, David, Belgrave South VIC 3160 (AU); KITCHEN, Barry, James, Bon Beach VIC 3196 (AU)
(74) Representative: Delorme, Nicolas
(86) International application number: PCT/AU2008/001458
(87) International publication number: WO 2009/043097

(56) References cited:
- JP-A- 2001 200 250
- JP-A- 2002 161 046
- JP-A- 2004 331 512
- JP-A- 2005 343 843
- JP-A- 2006 028 020
- US-A1- 2003 198 694
- US-A1- 2007 166 246
- JOAQUIM MAURÍCIO DUARTE-ALMEIDA ET AL: "Antioxidant Activity of Phenolics Compounds From Sugar Cane (Saccharum officinarum L.) Juice", PLANT FOODS FOR HUMAN NUTRITION, KLUWER ACADEMIC PUBLISHERS, DO, vol. 61, no. 4, 22 November 2006 (2006-11-22), pages 187-192, XP019453674, ISSN: 1573-9104, DOI: DOI:10.1007/S11130-006-0032-6
- NAKASONE YOKO ET AL: "Antioxidative compounds isolated from kokuto, non-centrifugal cane sugar", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 60, no. 10, 1 January 1996 (1996-01-01), pages 1714-1716, XP009142969, ISSN: 0916-8451
- PAYET ET AL.: 'Comparison of the Concentrations of Phenolic Constituents in Cane Sugar Manufacturing Products with Their Antioxidant Activities' J AGRIC FOOD CHEM vol. 54, 2006, pages 7270 - 7276, XP008133517
- PATENT ABSTRACTS OF JAPAN & JP 58 144382 A (AGENCY OF IND SCIENCE & TECHNOL) 27 August 1983

## Description

### Field of the invention

The present invention relates to preservatives and antimicrobial agents. In particular, the invention relates to preservatives derived from extracts derived from sugar cane which can be used to preserve food, cosmetics, pharmaceuticals and other similar compositions and to antimicrobial agents derived from extracts derived from sugar cane which can be used in oral hygiene products.

### Background of the invention

In this specification where a document, act or item of knowledge is referred to or discussed, this reference or discussion is not an admission that the document, act or item of knowledge or any combination thereof was at the priority date, publicly available, known to the public, part of common general knowledge; or known to be relevant to an attempt to solve any problem with which this specification is concerned.

### Preservatives

A preservative is a natural or synthetic chemical that is added to products such as foods, pharmaceuticals, biological samples, wood, etc. to prevent decomposition by microbial growth or by undesirable chemical changes.

Food preservation is the process of treating and handling food in a way that preserves its edibility and nutrition value. The main effort is to stop or greatly slow down spoilage to prevent foodborne illness (e.g. by salting, cooling, cooking). However some methods utilise benign bacteria, yeasts or fungi to add specific qualities and to preserve food (e.g. cheese, wine). While maintaining or creating nutritional value, texture and flavour is important in preserving its value as food; this is a culturally dependent determinant as what qualifies as food fit for humans in one culture may not qualify in another culture.

Preservation usually involves preventing the growth of bacteria, fungi and other microorganisms, as well as retarding the oxidation of fats which cause rancidity. It also includes processes to inhibit natural ageing and discolouration that can occur during food preparation such as the enzymatic browning reaction in apples which causes browning when apples are cut. Some preservation methods required the food to be sealed after treatment to prevent re-contamination with microbes; others, such as drying, allow food to be stored without any special containment for long periods.

Common methods of applying these processes include drying, spray drying, freeze drying, freezing, vacuum-packing, canning, preserving in syrup, sugar crystallisation, food irradiation, adding preservatives or inert gases such as carbon dioxide. Other methods that not only help to preserve food, but also add flavour, include pickling, salting, smoking, preserving in syrup or alcohol, sugar crystallisation and curing.

Preservative food additives can be used alone or in conjunction with other methods of food preservation. Preservatives may be anti-microbial preservatives, which inhibit the growth of bacteria and fungi, or antioxidants such as oxygen absorbers, which inhibit the oxidation of food constituents. Common anti-microbial preservatives include calcium propionate, sodium nitrate, sodium nitrite, sulfites (sulfur dioxide, sodium bisulfite, potassium hydrogen sulfite, etc.) and disodium EDTA. Common antioxidants include BHT (butylated hydroxy toluene) and BHA (butylated hydroxy anisole). Other preservatives include formaldehyde (usually in solution), glutaraldehyde (kills insects), ethanol and methylchloroisothiazolinone. The benefits and safety of many artificial food additives (including preservatives) are the subject of debate among academics and regulators specializing in food science and toxicology.

Antioxidants are used in a wide range of food products including, but not limited to, meat, poultry, fats, oils, margarines, fish, seafood and baked goods to inhibit the break-down of fats and oils or their oxidised products. The market is currently dominated by synthetic preservatives such as BHT and BHA. These are starting to be replaced by natural preservatives such as rosemary, tea extracts, tocopherol and ascorbate. However, the current natural preservatives can be expensive which limits their broader use in foods, leading to continued use of the synthetic preservatives.

Antimicrobials are also currently employed as preservatives within the food industry to extend product shelf life, improve product safety, maintain product quality, reduce processing costs and increase the ability to distribute products globally in complex supply chains. Due to consumer concern about the use of synthetic additives, the market for synthetic antimicrobials is declining and being replaced with natural antimicrobials.

Natural substances such as salt, sugar, vinegar, and diatomaceous earth are also used as traditional preservatives. Another group of preservatives targets enzymes in fruits and vegetables that continue to metabolize after they are cut. For instance, citric and ascorbic acids from lemon or other citrus juice can inhibit the action of the enzyme phenolase which turns surfaces of cut apples and potatoes brown.

### Rancidity

Rancidification is the decomposition of fats, oils and other lipids by hydrolysis or oxidation, or both. Hydrolysis will split fatty acid chains away from the glycerol backbone in glycerides. These free fatty acids can then undergo further auto-oxidation. Oxidation primarily occurs with unsaturated fats by a free radical-mediated process. These chemical processes can generate highly reactive molecules in rancid foods and oils, which are responsible for producing unpleasant and noxious odours and flavours. These chemical processes may also destroy nutrients in food. Under some conditions, rancidity, and the destruction of vitamins, occurs very quickly.

When a fatty substance is exposed to air, its unsaturated components are converted into hydroperoxides, which break down into volatile aldehydes, esters, alcohols, ketones, and hydrocarbons, some of which have disagreeable odours. Butter becomes rancid by the foregoing process and by hydrolysis, which liberates volatile and malodorous acids, particularly butyric acid. Saturated fats such as beef tallow are resistant to oxidation and seldom become rancid at ordinary temperatures.

Factors which accelerate fat oxidation include trace metals (iron, zinc, etc.), salt, light, water, bacteria, and moulds. Fat oxidation can be retarded by use of spices such as sage and rosemary, and by storing fats and oils in a cool, dark place with little exposure to oxygen or free-radicals, since heat and light accelerate the rate of reaction of fats with oxygen.

Antioxidants are often added to fat-containing foods in order to retard the development of rancidity due to oxidation. Natural antioxidants include flavonoids, polyphenols, ascorbic acid (vitamin C) and tocopherols (vitamin E). Synthetic antioxidants include BHA, BHT, propyl 3,4,5-trihydroxybenzoate (also known as propyl gallate) and ethoxyquin. The natural antioxidants tend to be short-lived, so synthetic antioxidants are used when a longer shelf-life is preferred. The effectiveness of water-soluble antioxidants is limited in preventing direct oxidation within fats, but is valuable in intercepting free-radicals that travel through the watery parts of foods.

### Natural antioxidants

Some modem synthetic preservatives have become controversial because they have been shown to cause respiratory or other health problems. Some studies point to synthetic preservatives and artificial coloring agents aggravating ADD & ADHD symptoms in those affected. Several major studies show academic performance increased and disciplinary problems decreased in large non-ADD student populations when artificial ingredients, including preservatives were eliminated from school food programs. Allergenic preservatives in food or medicine can cause anaphylactic shock in susceptible individuals, a condition which is often fatal within minutes without emergency treatment.

Accordingly, there is now a trend to use natural antioxidants. Plants contain phytochemicals, many of which are known to have antioxidant properties. Polyphenols is a generic name for a group of phytochemicals including flavonoids, anthocyanins and phenolic acids which occur naturally in a wide range of plants. Most are coloured and are responsible for the colors found in the fruits and in other parts of the plants. Their bioactivity is principally as an antioxidant which assists in protecting the plant from tissue damage and invasion by microorganisms.

The literature on plant phenolics is extensive and various polyphenols associated with different plant species have been well characterized and studied. The physiological effects of ingestion of many such polyphenols has also been examined clinically and they have been found not only to exhibit antioxidant activity but also anti-inflammatory and vasodilation properties.

Commonly consumed foods such as coffee, tea, cocoa (chocolate), red wine, berries (blue berries, blackberries, strawberries) and fruits (mangosteens, Noni, pomegranates, Acai, grapes) are now being shown and marketed for their high antioxidant levels and health promoting properties.

In general, plants and plant products have a much higher antioxidant content than do animal food products. Certain spices, berries, fruits, nuts, chocolate-containing products, vegetables and cereals are good sources of dietary antioxidants. Further, the drinks of coffee, green and black tea, red wine and various berry and fruit juices are good sources of antioxidants.

Antioxidant syrups and powders from many of the above sources are now being offered as food ingredients and additives for use in a wide range of food systems. High polyphenol antioxidant syrups and powders (eg grape derived Vinlife from Tarac Technologies in South Australia and Polyphenolics from California, USA) are finding their way into products such as chocolate (Cocoa Farm, Melbourne Australia), cocoa beverages, teas and ice creams (Wendy's Vinlife Ice Cream, Australia). However, these food ingredients are usually being used to increase the antioxidant content of the food to provide beneficial health effects and not as food preservatives.

Sugar cane is known to contain polyphenols and other phytochemicals having beneficial properties. Examples of publications regarding these properties include international patent application nos WO 2005/117608, PCT/AU2006/000769 and PCT/AU2007/001382. However, these documents do not disclose the use of low colour extracts of sugar cane as food preservatives.

The antioxidant activity of a number of sugar cane manufacturing products is measured in Payet et al, "Comparison of the Concentrations of Phenolic Constituents in Cane Sugar Manufacturing Products with their Antioxidant Activities" J Agric Food Chem, 2006, 54, 7270-7276. The document speculates that the antioxidant activity may be due to the effect of Maillard reaction products and the large increase in colorant. Preservatives which have a high colour would have limited use as a food additive. There is no disclosure of the use of low colour extracts of sugar cane as food preservatives.

Japanese patent publication no 2001-112439 discloses that an extract from brown sugar which contains melanoidins has an antioxidant effect, can be used to reduce body fat and improve skin condition. However, melanoidins are brown, high molecular weight heterogeneous polymers that are formed when sugars and amino acids combine (through a Maillard reaction) at high temperatures and low water activity. The extract would therefore have a high colour which would limit its use as a food additive. The extract may also have a strong flavour which would further limit its use as a food additive. There is no disclosure of the use of low colour extracts of sugar cane as food preservatives

Japanese patent publication no 2002-161046 discloses that polyphenols can be extracted from sugar cane ear which have antioxidant properties. There is no disclosure of the use of low colour extracts of sugar cane as food preservatives.

Japanese patent publication no 2001-200250 discloses that extracts of sugar cane have antioxidant properties which can be used as food preservatives. However, there is no disclosure of the use of low colour extracts of sugar cane as food preservatives.

Whilst natural antioxidants, such as rosemary, tea extracts, tocopherol and ascorbate, are currently being used to preserve food. These natural antioxidants tend to be expensive. Tocopherol also has practical issues because it is hydrophobic. Ascorbate has practical issues since it adds an acidic taste to foods.

### Antimicrobial agents

Agents that kill or inhibit microorganisms may be classified as disinfectants, antiseptics or antibiotics. Antibiotics are molecules that are produced by one microorganism that kill (bacteriocidal) or inhibit (bacteriostatic) other microorganisms. Antiseptics and disinfectants are commercially prepared chemicals and the distinction between them is that antiseptics can be exposed to mucosal surfaces for at least a short time and disinfectants should not as they could impart harm.

The ADA's Council on Scientific Affairs has highlighted the oral health benefits of other ADA-Accepted products such as antimicrobial mouth rinses and toothpastes that can help prevent and reduce plaque and gingivitis, and fluoride mouth rinses that can provide extra protection against tooth decay over that provided by fluoride toothpaste alone.

Rinses are generally classified by the U.S. Food and Drug Administration (FDA) as either: cosmetic, therapeutic, or a combination of the two. Cosmetic rinses are commercial over-the-counter (OTC) products that help remove oral debris before or after brushing, temporarily suppress bad breath, diminish bacteria in the mouth and refresh the mouth with a pleasant taste. Therapeutic rinses have the benefits of their cosmetic counterparts, but also contain an added active ingredient that helps protect against some oral diseases. Therapeutic rinses are regulated by the FDA and are voluntarily approved by the American Dental Association (ADA).

Most mouth rinses are, at the very least, effective oral antiseptics that freshen the mouth and curb bad breath for up to three hours. Their success in preventing tooth decay, gingivitis (inflammation of the gingival gum tissue) and periodontal disease is limited, however. Therapeutic anticavity rinses with fluoride, however, have been clinically proven to fight up to 50 percent more of the bacteria that cause cavities. Most anticavity rinses contain sodium fluoride, which if taken excessively or swallowed, can lead over time to fluoride toxicity.

Most over-the-counter mouth rinses contain five standard components:
- an active bacteria- fighting ingredient such as quaternary ammonium compounds, boric and benzoic acid, phenolic compounds;
- a flavouring agent such as saccharin or glycerine;
- astringents like zinc chloride to provide a pleasant-tasting sensation and shrink tissues;
- ethyl alcohol, ranging from 18 to 26 percent; and
- water.

Rinses can also contain buffers to reduce acidity, dissolve mucous films and alleviate soft tissue pain. Anticavity rinses usually contain 0.05 percent sodium fluoride, or 0.1 percent stannous fluoride, as approved by the FDA.

Active ingredients in antiplaque rinses vary. Certain rinses contain chlorhexidine (the most effective plaque-fighting drug yet tested, available only by prescription), heavy metal salts or herbal extracts like sanguinaria, derived from the bloodroot plant.

Antimicrobial mouth rinses and toothpastes reduce the bacterial count and inhibit the bacterial activity in dental plaque that can cause gingivitis, an early, reversible form of periodontal (gum) disease. ADA-Accepted antimicrobial mouth rinses and toothpastes have substantiated these claims by demonstrating significant reductions in plaque and gingivitis. The concentration of the active substance in the antimicrobial mouthwashes is often well in excess of the minimum inhibitory concentrations determined providing a rapid lethal effect on the bacteria which can make them useful in infectious conditions.

However, many people are wary of using commercial mouthwash products because of concerns regarding toxicity and damage to teeth. Natural alternatives include a saline solution rinse and a sodium bicarbonate solution rinse.

There is thus a need for an alternative source of natural preservatives and antimicrobial agents.

### Summary of the invention

Molasses and other products of the sugar cane processing are complex mixtures of phytochemicals which typically comprise polyphenols, polysaccharides, peptides and proteins, minerals, organic acids, and mono and disaccharides. The present invention provides low colour extracts derived from sugar cane which have food preservative and antimicrobial agent properties. In particular, it has surprisingly been found that low colour extracts derived from sugar cane have a high antioxidant activity making them useful to inhibit the break-down of fats and oils or their oxidised products and as antimicrobial agents.

According to a first aspect of the invention, there is provided a preservative comprising a low colour polyphenol enriched molasses extract derived from sugar cane which has a high antioxidant activity, wherein the extract has a polyphenol content of at least about 50 µg/ml catechin equivalents and the extract is low in colour compared to molasses.

According to a second aspect of the invention, there is provided a method of preserving food, cosmetics or pharmaceuticals comprising the step of adding to the food, cosmetic or pharmaceutical an effective preserving amount of a low colour extract derived from sugar cane which has a high antioxidant activity, as described above.

The low colour extract used in this aspect of the invention provides both antioxidant and antimicrobial characteristics. Previously, different preservatives would be used to obtain each of these effects

The term "low colour" when used herein refers to an extract derived from sugarcane which has an absorbance value of less than or equal to about 0.010 when measured at 750 nm.

The term "high antioxidant" when used herein refers to an extract derived from sugarcane which has an antioxidant level of at least about 50 µg/ml catechin equivalents when tested as described in Example 4. Preferably, the extract derived from sugarcane has an antioxidant level of 0.99 to 6 g/l catechin equivalents; more preferably 4 to 5 g/l catechin equivalents.

According to a third aspect of the invention, there is provided use of a low colour extract derived from sugar cane which has a high antioxidant activity as described above as an antioxidant in food, cosmetics or pharmaceuticals.

This aspect of the invention uses an extract which is a cost efficient, rich source of antioxidants and which can be used to replace the expensive synthetic antioxidants currently employed by the food industry.

According to a fourth aspect of the invention, there is provided use of a low colour extract derived from sugar cane which has a high antioxidant activity, as described above, as an antimicrobial in food, cosmetics or pharmaceuticals.

This aspect of the invention uses an extract which is high in polyphenols and may be used to meet the increasing demand for natural antimicrobials by the global food industry. In addition, the extracts according to the invention are likely to have a price advantage over currently employed antimicrobials.

The term "effective preserving amount" means an amount which will minimise or substantially inhibit the rancidification of fats and oils (antioxidant properties) and/or minimise or substantially inhibit microbial growth (antimicrobial). The specific amount used will depend on the particular composition and desired shelf life. Typically, the amounts used will be in the range of 0.0001 to 5% by weight of the total composition, more typically 0.01 to 2.5%.

The preservative of the present invention may be incorporated directly and without further modification into food, cosmetics or pharmaceuticals (enteral and parenteral products) by techniques such as mixing, infusion, injection, blending, dispersing, conching, emulsifying, immersion, spraying, agglomeration and kneading. According to a fifth aspect of the invention, there is provided a preservative for use in improving oral hygiene and/or inhibiting, treating and/or preventing the formation of dental caries, wherein a therapeutically effective amount of a low colour extract derived from sugar cane which has a high antioxidant activity as described above, is added to an oral hygiene product.

The term "therapeutically effective amount" as used herein refers to an amount which will minimise or substantially inhibit microbial growth and/or kill microbes in the oral cavity. Typically, the amounts used will be in the range of 0.0001 to 5% by weight of the total composition, more typically 0.01 to 2.5%.

The term "oral hygiene products" as used herein includes, but is not limited to, teeth cleaning products such as toothpaste, mouthwash (mouth rinses), and chewing gum.

According to a sixth aspect of the invention, there is provided use of a therapeutically effective amount of a low colour extract derived from sugar cane which has a high antioxidant activity as described above, in the preparation of an oral hygiene product to improve oral hygiene and/or inhibit, treat and/or prevent the formation of dental caries.

The extract can be derived from any product derived from sugar cane including the sugar cane milling process, the sugar cane refining process to make sugar, and other processes using sugar cane products such as the manufacture of ethanol from molasses as part of the manufacture of rum. The extract can be derived from the raw materials, in-process products, by-products, final products and waste streams. For example, the sugar cane extract may be derived from the feed stream of raw sugar cane juice, clarified juice and concentrated juice syrup, treacle, molasses (obtained from a primary mill or refinery), golden syrup, brown sugar, bagasse, biodunder, field trash, growing tips, pulp, cane strippings, pith, regeneration extracts (neutralised and non-neutralised) and mill mud. In accordance with the invention, the extract is derived from molasses.

The physical characteristics of the extracts for use in the present invention will depend on their overall chemical composition. Depending on the processing methods applied, the extracts may be concentrated by evaporation generating a syrup, or alternatively, the extract could be fully dried to produce a powder. This ability to prepare extracts having different physical properties increases the commercial utility of the extracts. Depending on their physical characteristics and chemical composition the extracts will be suitable for various uses. For example, the requirements for the food industry could be very different to the requirements for the cosmetic industry.

As used herein, the term "food" includes any edible product, such as but not limited to confectioneries, supplements, snacks (sweet and savoury), cocoa & coffee-containing foods, flavours, beverages (including instant beverages, pre-mixes), nutriceuticals, dietary supplements and formulations including supplements used in animal health and nutrition, dairy products eg: milk, yogurt, ice-cream, baked products, and food seasonings, and animal feeds.

The preservative of the present invention may be incorporated into foods including, without limitation, the following:
- Dairy Products--such as cheeses, butter, milk and other dairy beverages, spreads and dairy mixes, ice cream and yoghurt;
- Fat-Based Products--such as margarines, spreads, mayonnaise, shortenings, cooking and frying oils and dressings;
- Cereal-Based Products--comprising grains (for example, bread and pastas) whether these goods are cooked, baked or otherwise processed;
- Confectioneries--such as chocolate, candies, chewing gum, desserts, non-dairy toppings, sorbets, icings and other fillings;
- Sports nutrition products including powders, pre-mixes, juices, energy bars, isotonic drinks and gelatine, starch based or pectin jellies;
- Beverages--whether hot or cold (coffee, tea, cocoa, cereal, chicory and other plant extract based beverages), alcoholic or non-alcoholic and including colas and other soft drinks, juice drinks, dietary supplement, instant pre-mixes and meal replacement drinks; and
- Miscellaneous Products--including eggs and egg products, processed foods such as soups, pre-prepared pastas.

### Method of Preparing the Extracts used in the Present Invention

The extracts used in the present invention are derived from sugar cane product, preferably molasses from the cane sugar refining processes. The extract may be obtained from the sugar cane product by various methods, or combinations of methods, such as:
- solvent and counter-current extraction using non-aqueous or aqueous solvents;
- separation of components falling with in a specific molecular weight range by size exclusion processing methods such as gel permeation chromatography or ultrafiltration; and
- separation of the low and high molecular weight components using chromatographic techniques or combinations of techniques such as ion exchange chromatography, hydrophobic chromatography and ion exchange chromatography using fractional elution by stepwise increase in pH or with solvents such as ethanol.

The extracts may be further processed by standard techniques such as microfiltration, reverse osmosis, vacuum evaporation and freeze drying, spray drying and tunnel drying.

Examples of methods to prepare the extracts are disclosed in international patent application nos WO 2005/117608 and PCT/AU2007/001382.

### Brief description of the figures

Figure 1 shows the gel filtration of molasses on Bio-Gel P-2 showing A420 and antioxidant activity for Run 3 from Example 1.
Figure 2 shows the gel filtration of molasses on Bio-Gel P-2 showing A420 and total phenolics for Run 3 from Example 1.
Figure 3 shows the gel filtration of molasses on Bio-Gel P-2 showing A420 and sucrose for Run 3 from Example 1.
Figure 4 shows the gel filtration of molasses on Bio-Gel P-2 showing A420 and glucose + fructose for Run 3 from Example 1.
Figure 5 is a photograph of the fractionation of molasses obtained on Bio-Gel P-2 (Pools 1 - 5 from Example 1).
Figure 6 shows the gel filtration of molasses on Bio-Gel P-2 showing A420 profiles using formate/acetonitrile buffer pH 5.0 and Tris HCl buffer pH 7.5 from Example 1.
Figure 7 shows the 96-well plate map from Example 4.
Figure 8 shows the raw absorbance values, A₇₅₀ from Example 4.
Figure 9 shows the corrected absorbance values from Example 4
Figure 10 shows the map of 96 well plate of molasses feedstock dilutions and fractions 3-23 from Example 4.
Figure 11 shows the absorbance values at 750 nm of the map in Figure 11.
Figure 12 shows a chromatogram from Example 4.
Figure 13 shows the catechin standard curve from Example 4.
Figure 14 shows the total polyphenols in catechin equivalence of each fraction from fraction 3-26, with error of ±2SD from Example 4.
Figure 15 shows the total polyphenols in catechin equivalence of each plate, showing fractions 3-26, with error of ±2SD from Example 4.
Figure 16 shows the colour fractions collected from Example 4.

### Examples

Various embodiments/aspects of the invention will now be described with reference to the following non-limiting examples.

### List of Abbreviations

- BDL: Below Detectable Limit
- CE: Catechin Equivalents
- DW: Dry Weight
- GAE: Gallic Acid Equivalents
- GI: Glycaemic Index
- IC or ICUMSA: International Commission for Uniform Methods of Sugar Analysis
- MF: Microfiltration
- N/D: Not Detected
- N/T: Not Tested

### Example 1

This example investigates the use of gel permeation to produce extracts which can be used in the invention.

### Methods

Preparative gel filtration on a Bio-Gel P-2 column was used to fractionate diluted molasses (50% w/v) in the molecular weight range of 100 to 1800 daltons (Da). Five molecular weight fractions from six chromatography runs were pooled and freeze dried. The fractions were analysed for antioxidant activity, total phenolics, HPLC profile and sugars.

The molasses was diluted to 50% (w/v) in gel filtration buffer (20 mM ammonium formate pH 5.0 containing 10% acetonitrile) and centrifuged at 6000 *g* for 1 hour at 10°C. The supernatant was filtered through a 1.6 µm GF/A filter (Whatman) and frozen in 30-ml aliquots at -80°C for use in gel filtration chromatography.

### Gel filtration

A glass column (26 mm x 1000 mm) was packed with Bio-Gel P-2 (BioRad, USA) to a bed height of 910 mm at a flow rate of 60 ml/h. The bed was equilibrated at 30 ml/h at room temperature in 20 mM ammonium formate buffer pH 5.0 containing 10% acetonitrile. Diluted molasses (20 ml of 50% w/v) was applied to the column and 5-ml fractions were collected. Six gel filtration runs were carried out on a total of 120 ml of diluted molasses. Fractions from the first three runs were analysed for colour (A₄₂₀), total phenolics and antioxidant activity. For the last three runs antioxidant assays were omitted. Fraction volumes were determined gravimetrically by weighing approximately 20 tubes per run and determining the average fraction volume using a density of 1 g/ml.

The gel filtration column was calibrated with three standards: sucrose (364 kDa), NADH (663 kDa) and vitamin B12 (1355 kDa). The distribution coefficient (KDa) for each standard was calculated as KDa = Ve-Vo/Vt-Vo. The void volume was determined with bovine serum albumin. The fractionation range of Bio-Gel P-2 is 100-1800 Da (BioRad).

*Lyophilised bulk fractions:* For each ,gel filtration run, individual fractions were pooled into five major fractions according to profiles of colour (A₄₂₀), total phenolics and antioxidants. The pooled fractions for each run are shown in Table 1.

**Table 1: List of Pooled Fractions for each Run**

| **Run No** | **Fraction volume (ml)** | **Pool 1** | **Pool 2** | **Pool 3** | **Pool 4** | **Pool 5** |
|---|---|---|---|---|---|---|
| 1 | 4.88 | 34-50 | 51-66 | 67-82 | 83-100 | 101-120 |
| 2 | 4.74 | 35-54 | 55-72 | 73-83 | 84-104 | 105-120 |
| 3 | 4.95 | 35-52 | 53-68 | 69-79 | 80-100 | 101-120 |
| 4 | 4.69 | 35-53 | 54-71 | 72-81 | 82-100 | 101-120 |
| 5 | 4.52 | 37-56 | 57-76 | 77-87 | 88-103 | 104-120 |
| 6 | 4.71 | 35-51 | 52-71 | 72-81 | 82-99 | 100-120 |
| Total vol. (ml) | | 447 | 435 | 270 | 452 | 470 |

After six gel filtration runs, the six samples within each pool (Pools 1-5) were combined. A 10-ml sample was taken from each final pool and the remainder freeze dried.

*Colour (A₄₂₀):* Gel filtration fractions were diluted with ultrapure water (Arium Model 611, Sartorius) and absorbance was read at 420 nm on a Heliosλ (Unicam) spectrophotometer.

*Total phenolics:* Total phenolics were determined by a Folin-Ciocalteu colorimetric procedure (Kim *et al.,* 2003). To 50 µL of diluted sample in a 75-mm test tube was added 650 µL of deionised water. Undiluted Folin-Ciocalteu reagent (50 µL) was added to each tube. The solution was mixed and allowed to stand for 5 min at room temperature. Finally 500 µL of 7% Na₂CO₃ was mixed with the reaction solution and the absorbance at 750 nm was read after 90 min at room temperature. The total phenolics content was expressed in µg catechin equivalents per ml of undiluted sample. Catechin standards were prepared in the range of 0-250 µg/ml.

*Antioxidant activity:* Initially, a substrate containing equal volumes of 14 mM ABTS (2, 2'-azinobis (3-ethylbenzothiazoline-6-sulfonic acid) diammonium salt) and 4.9 mM potassium persulphate was prepared and stored overnight in the dark at room temperature. Prior to the assay, this solution was diluted about 60-fold with ultrapure water and adjusted to give an absorbance at 734 nm of 0.99-1.01. ABTS substrate (1 ml) was preincubated in 75-mm test tubes at 26°C for 5 min in a water bath ant 50 uL of sample or standard was added. The solution was mixed and held at 26°C for 45 min when absorbance was measured at 734 nm. Antioxidant activity was expressed in µg gallic acid equivalents per ml of undiluted sample. Gallic acid standards were prepared in the range 0-25 µg/ml.

*RP-HPLC profiles:* Qualitative fingerprints of molasses extracts were obtained on a Shimadzu system equipped with a system controller (Model SCL-10AVP), dual pumps (Model LC10-AD), photo diode array (PDA) detector (Model SPD-M10AVP) and Class Vp version 6.14 software for data acquisition and analysis. Samples (10 µL) were eluted at 30°C on a 30 x 4.6 mm Luna 3 µm C18(2) column (Phenomenex). The flow rate was 1.5 ml/min. Mobile phases were: phase A, 0.1% (v/v) trifluoroacetic acid (TFA) in water and phase B, 60% acetonitrile in 0.085% TFA. The gradient profile was 5-35%B for 12 min; 35-100%B for 1 min and 100%B for 3 min, 100-5%B for 0.3 min and 5%B reequilibration for 4.7 min. Eluted peaks were detected by a PDA detector measuring the absorbance spectrum from 200-400 nm at 4 nm wavelength steps and individual channels at 214, 254, 280, 340 and 400 nm, with the 214 nm chromatogram routinely reported. Gel filtration samples were prepared from five lyophilised pools and contained equal concentrations of total phenolics (1 mg catechin equivalents per ml). The sample of molasses used for gel filtration contained 2 mg CE/ml.

*Sugar analysis:* Mono- and disaccharides were analysed by reversed-phase HPLC using a Shimadzu system fitted with a system controller (Model SCL-10AVP), pump (Model LC-10ADVP), refractive index detector (Model RID-14A) and Class Vp 6.12 software. Samples (10 uL) were injected into a 5-µm LC-NH2 Supelcosil column (250 mm x 4.6 mm, Phenomenex) operated at 40°C. The mobile phase was 85% acetonitrile and the flow rate was 1 ml/min. Samples were eluted isocratically for 20 min and were analysed in duplicate. Standard curves for glucose, fructose and sucrose were prepared in the range 0.3 to 1.2 mg/ml, using four standard solutions containing the same gravimetric concentrations of the respective sugars. Triplicate injections were made for each standard solution.

*SDS-PAGE.* Electrophoresis by SDS-PAGE was performed on 12% acrylamide gels using the mini-Protean II slab-gel system (BioRad). Lyophilised samples from gel filtration were dissolved in water (200 mg/ml) and 30 ul was digested in an equal volume of loading buffer. A volume of 15 ul (1.5 mg of solids) was loaded onto the gel. Electrophoresis was stopped when the bromophenol blue dye front reached the bottom of the gel. The gel was stained in 0.25% Coomassie Blue and scanned on a desktop scanner (Scanjet 5400C, Hewlett Packard).

### Results

### Calibration of Bio-Gel P-2:

A calibration curve for determining molecular weights on the Bio-Gel P-2 column was prepared.

### Gel filtration profiles

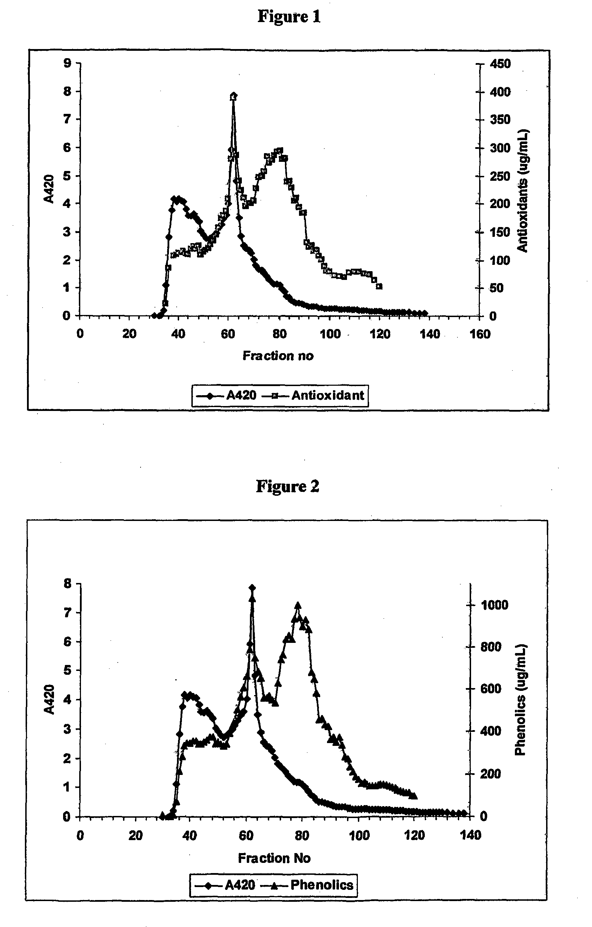

The gel filtration profiles for colour (A₄₂₀), antioxidants and total phenolics from molasses (Run 3) are shown in Figures 1 and 2. The A₄₂₀ colour profile showed a peak near the void volume of the column and a sharp peak at fraction 62 (MW 832 Da). The absorbance then decreased gradually to baseline. Profiles of antioxidants and total phenolics closely coincided with each other. The first two antioxidant/phenolic peaks coeluted with the A₄₂₀ peaks. However, a broad antioxidant/phenolic peak at fraction 80 (MW 352) did not correspond to a colour peak. This peak, comprising fractions 69-100 has a molecular weight range of 135-599 Da and may be a mixture of low colour flavonoids and polyphenolic acids.

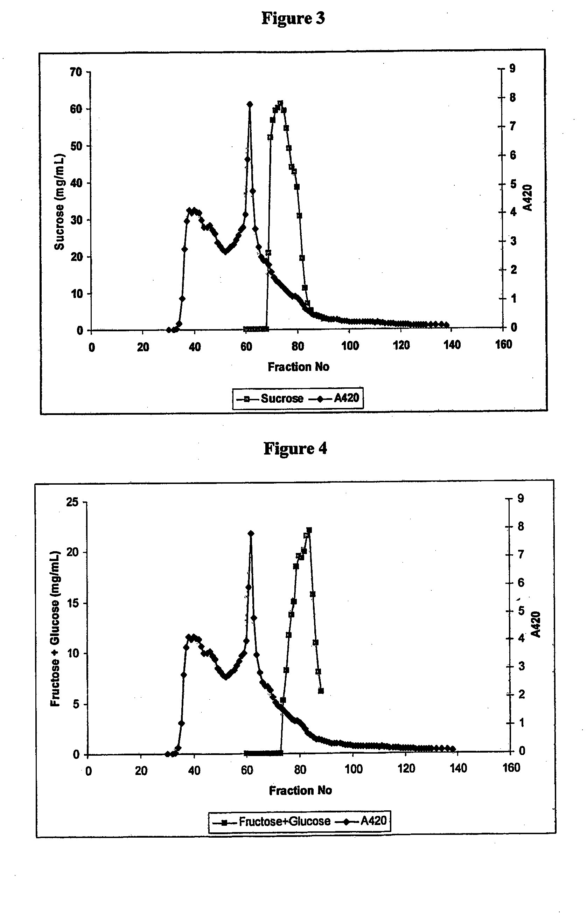

Profiles of sucrose and monosaccharides (glucose + fructose) are shown in Figures 3 and 4 respectively. The column was capable of partially resolving sucrose and monosaccharides. Sucrose was eluted on the leading edge of the antioxidant peak (pre fraction 80) and monosaccharides on the tailing edge (post fraction 80). Hence the low colour antioxidant peak contains all the simple sugars of molasses.

For membrane filtration applications, where a low colour antioxidant product is required, it would be necessary to target the molecular weight region below 600 Da. Separation of the antioxidants from the sugars should be possible by ion exclusion chromatography.

### Bulk gel filtration pools

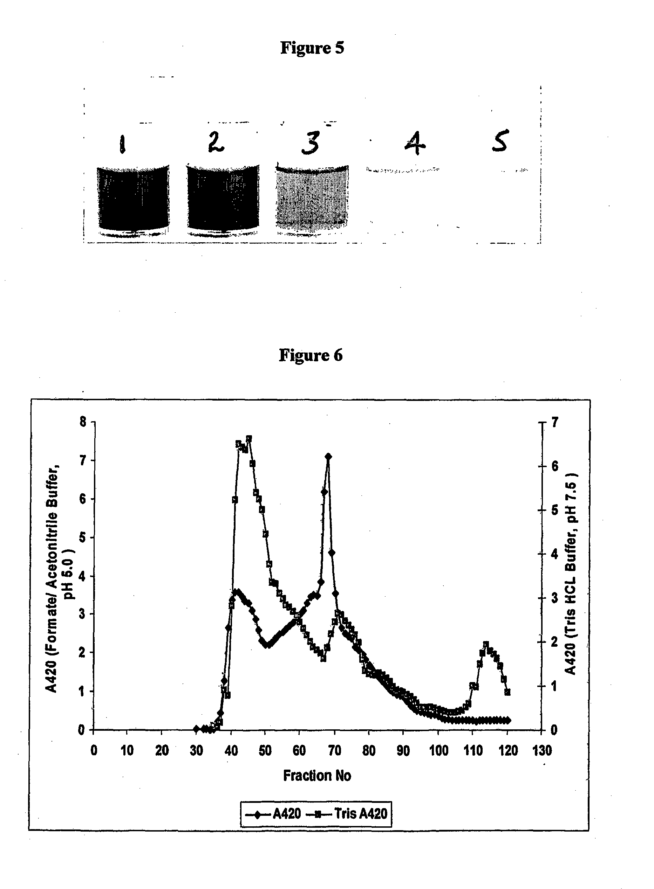

The five pools for each of the six gel filtration runs were thawed and combined before freeze drying. Figure 5 shows the colours of the combined pools (Pools 1-5) prior to freeze drying. Pools 1 and 2 were both very dark; Pool 1 was slightly turbid and Pool 2 was translucent. Pools 3-5 showed decreasing colour from light brown to pale yellow.

Table 2 shows the composition of the combined pools before freeze drying and the average molecular weight range for each pool. From the mass calculations, the low colour antioxidant/phenolic peak (Figures 1 and 2) contained 49% of the antioxidant activity and 50% of the phenolics, respectively. The dark-coloured peak eluting at the void volume (Pool 1) contained 14% of the antioxidant activity, and the dark-coloured sharp peak (Pool 2) contained 28%. Recovery of antioxidant activity from the column was 70%.

**Table 2: Composition of combined pools from gel filtration on Bio-Gel P-2 prior to freeze drying.**

| **Component** | **Pool 1** | **Pool 2** | **Pool 3** | **Pool 4** | **Pool 5** |
|---|---|---|---|---|---|
| Volume (ml) | 437 | 425 | 260 | 442 | 460 |
| Total solids (g/100 ml) | 0.56 | 1.36 | 7.3 | 3.24 | 0.15 |
| Total phenolics (µg CE/ml) | 324 | 555 | 761 | 550 | 141 |
| Antioxidant activity (µg GAE/ml) | 92 | 183 | 229 | 173 | 49 |
| Fructose (mg/ml) | BDL | BDL | 4.0 | 5.1 | BDL |
| Glucose (mg/ml) | BDL | BDL | 5.1 | 4.2 | BDL |
| Sucrose (mg/ml) | BDL | 0.70 | 50 | 12 | BDL |
| Total solids (g) | 2.45 | 5.78 | 18.98 | 14.32 | 0.69 |
| Total phenolics (mg CE) | 142 | 236 | 198 | 243 | 65 |
| Antioxidants (mg GAE) | 40 | 78 | 60 | 76 | 23 |
| Average molecular wt range | >1800-1444 | 1377-636 | 604-373 | 356-156 | 150-65 |

Composition of load molasses: Total solids = 35.6 g/100 ml; A₄₂₀ = 43.7;
Total phenolics = 10340 µg/ml; Antioxidant activity = 3390 µg/ml.
Total volume of molasses for 6 runs = 120 ml.

Table 3 shows the composition of combined pools after freeze drying. With respect to physical properties, Pool 1 was a fluffy product and differed considerably from Pool 2 which had a hard crunchy texture. Pools 3 and 4 were crunchy and hygroscopic, and contained 71 % and 64% sugars, respectively. Pool 5 was dark and sticky and was difficult to remove from the drying tray resulting in significant loss of product. On a solids basis (mg GAE/g of solids), there was a significant loss in antioxidant activity on freeze drying of Pool 2 (26%) and Pool 5 (34%).

**Table 3: Composition of combined pools from gel filtration on Bio-Gel P-2 after freeze drying**

| **Component** | **Pool 1** | **Pool 2** | **Pool 3** | **Pool 4** | **Pool 5** |
|---|---|---|---|---|---|
| Weight of dried product (g) | 2.62 | 5.23 | 18.17 | 13.74 | 0.5 |
| Total phenolics (g/100 g) | 5.60 | 4.1 | 0.99 | 1.70 | 5.42 |
| Antioxidant activity (g/100 g) | 1.6 | 0.99 | 0.29 | 0.55 | 2.1 |
| Fructose (g/100 g) | BDL | BDL | 5.0 | 16 | BDL |
| Glucose (g/100 g) | BDL | BDL | 7.1 | 14 | BDL |
| Sucrose (g/100 g) | BDL | 6.7 | 59 | 34 | BDL |
| Total phenolics (mg CE) | 147 | 214 | 180 | 234 | 27 |
| Antioxidants (mg GAE) | 42 | 52 | 53 | 76 | 11 |
| Texture | Fluffy | Crunchy | Crunchy | Crunchy | Tacky |
| Colour | Black | Black | Light brown | Light brown | Black |

Composition of load molasses: Total solids = 35.6,g/100 ml; A₄₂₀ = 43.7;
Total phenolics = 10340 µg/ml; Antioxidant activity = 3390 µg/ml.
Total volume of molasses for 6 runs =120 ml.

*RP-HPLC profiles:* Reversed phase HPLC profiles of lyophilised gel filtration pools (profiles b-f) were examined. There were notable differences between all profiles which could be used to characterise the pools. Pool 1 showed a gradual rising profile with only one minor peak. Presumably this sample contains heterogeneous polymeric material which could not be resolved to individual peaks by the HPLC column. Pool 2 represents the molecular weight range 636-1377 Da and includes the sharp peak of dark brown material. This pool showed the most hydrophilic material eluting at less than 1 min, and a number of well-resolved peaks on the gradient. Pools 3 and 4 represent the low-colour antioxidant peak and showed considerable differences between their respective profiles. Pool 5 showed a range of peaks which could represent low molecular weight phenolic acids and a higher molecular weight compounds that have been weakly bound to the column and were not eluted according to their molecular weights. The proportion of hydrophilic material in this pool was low resulting in greater peak heights in the hydrophobic region of the profile. The molasses load sample enables some peaks to be matched up to certain molecular weight ranges in the pools, as well as showing which molasses peaks are weakly bound to the gel and eluted in Pool 5. All samples exhibited a significant peak at 14.5 min which is not relevant to the chromatography, and represents an acetonitrile flush to remove all bound material from the column at the end of the run. Interestingly, this peak which represents the more hydrophobic compounds in molasses, decreased with molecular weight of the pools.

*SDS-PAGE:* Denaturing electrophoresis of the lyophilised pools was used to detect protein material in the extracts. No protein bands were apparent above 14 kDa in the extracts. In Pool 1 (Lane 2) light staining was observed close to the dye front, but it is uncertain if this is stained protein or residual Coomassie Blue from an uneven dye front. Detection of low molecular weight polypeptides (<10 kDa) would require a 16% gel with a Tris-Tricine buffer.

### Conclusion

The gel filtration profiles showed that dark molasses colorants measured at 420 nm were eluted in the void volume of the column (>1800 Da) and at 832 Da. Antioxidant activity and total phenolics co-eluted with these two colour peaks. A broad antioxidant/phenolic peak was eluted between 135 and 599 kDa, but was not associated with a colour peak. This antioxidant peak contained all the sucrose and monosaccharides. It comprised 49% of the eluted antioxidant activity and 50% of the total phenolics. Consequently, removal of the dark colorants of molasses would approximately halve the antioxidant activity of the product The dark-coloured polymeric material eluting near the void volume comprised 14% of the eluted antioxidant activity.

The quantities of lyophilised gel filtration pools varied from 0.5 g to 18 g, with high masses obtained for the two pools containing sugars. Recovery of antioxidant activity was greater than 92% in three of the lyophilised pools, but significant losses in antioxidant activity were found in Pools 2 and 5.

HPLC fingerprints of the lyophilised pools showed some distinct differences which could be used to characterise the samples. Protein analysis by denaturing polyacrylamide gel electrophoresis showed an absence of protein material above 14 kDa in all lyophilised samples. Pool 1 showed a trace of a protein stain near the dye front. This could be bound protein associated with hydrolysable tannins

The lyophilised samples will be analysed subsequently for polysaccharides and polyphenol characterisation, and for their ability to inhibit gut enzymes.

The example demonstrates that the colour profiles obtained by the gel filtration of molasses were dependent on the pH and/or composition of the buffer and that a less coloured high antioxidant extract according to the invention can be produced. At pH 7.5, most of the dark colour was eluted at the void volume, while at pH 5.0, a second dark-coloured peak was observed at lower molecular weight. The pH 5.0 buffer contained 10% acetonitrile which could have contributed to a change in the permeation properties of the gel.

Such a lower colour high antioxidant extract will be useful as an additive to foods to reduce GI, reduce carcinogenicity, change body composition and be useful as an antioxidant or antimicrobial without interference with the colour or organoleptic properties of the food. Further, a lower colour extract according to the invention is useful in pharmaceutical applications, especially where colour and bitterness are important issues.

### Example 2

This example further investigates the polyphenols composition of pools 1 to 4 from Example 1. This example also demonstrates the polyphenols content of an extract of dunder.

### Methods

*Solvent extraction:* An aliquot of each sample (∼200 mg) was dissolved in water (10 ml) that had been acidified (pH 1.6). The mixtures were then extracted with ethyl acetate (3 x 15 ml), the solvent was evaporated under vacuum (40°C) and the mixtures were reconstituted in aqueous methanol (1:1, 2 ml) before subjected to HPLC analysis.

*HPLC analysis:* HPLC was carried out using a Shimadzu system equipped with two highpressure LC-10ADVP pumps, a SIL-10ADVP autosampler (250 µl sampling loop), a CTO-1-ADVP column oven and a SPD-M10ADVP photodiode array detector (Shimadzu Inc., Rydalmere, NSW, Australia). The column used for the separation of the polyphenols was a Luna C₁₈, (4.6 mm i.d. x 250 mm length, 5 µm particle size, Phenomenex, Lane Cove, NSW, Australia). The mobile phase used for the separation were 2% acetic acid in water (A) and 0.5% acetic acid in acetonitrile:water (1:1) (B) under a flow rate of 1 ml.min⁻¹. Analytes were eluted using a linear gradient: 10 - 100% B over 59 min. and remained at 100% B for another 5 min. Detection was carried out at 280, 320 and 370 nm. Analytes were identified by comparison of their elution time (and characteristic UV profile) with those of authentic standards (Sigma-Aldrich, Castle-Hill, NSW, Australia).

### Results and Discussion

Each compound was quantified based on the maximum UV absorbance with respect to one of the three specified wavelengths (280 nm, 320 nm and 370 nm). Quantification based on these wavelengths was obtained from calibration curves of syringic acid, p-coumaric acid and quercetin, respectively. The levels of eight compounds identified in the samples by HPLC-DAD are listed below (Table 4).

**Table 4: Concentrations of polyphenolic compounds found in the five molasses samples as detected by HPLC-DAD.**

| **Concentration (mg/kg)** | | | | | |
|---|---|---|---|---|---|
| **Target Compounds** | **Dunder** | **Pool 1** | **Pool 2** | **Pool 3** | **Pool 4** |
| Cinnamic acid | 473.6 | N/D | N/D | 2.5 | 6.2 |
| Epicatechin | N/D | 3.1 | 6.1 | 69.1 | 268.2 |
| Myrcetin | N/D | N/D | N/D | 0.9 | 62.9 |
| Syringic acid | 799.7 | 2.4 | 5.9 | 68.3 | 261.9 |
| Vanillic acid | N/D | n/d | 3.5 | 7.1 | 82.9 |
| *p*-hydroxybenzoic acid | N/D | N/D | N/D | N/D | 23.4 |
| Apigenin | 11.8 | N/D | N/D | N/D | N/D |
| Ferulic acid | N/D | N/D | N/D | N/D | 1.9 |

All samples contained different levels of syringic acid, the predominant compound in most samples. Epicatechin was found to be present in most samples.

**Table 5. Concentration of phenolic compounds at each wavelength for the 4 samples as detected by HPLC-DAD.**

| **Wavelength** | **Concentration (mg/kg)** | | | | |
|---|---|---|---|---|---|
| | **Dunder** | **Pool 1** | **Pool 2** | **Pool 3** | **Pool 4** |
| 280 nm | 4176.6 | 68.7 | 460.4 | 341.0 | 1277.8 |
| 320 nm | 406.3 | 36.3 | 64.3 | 58.2 | 199.8 |
| 370 nm | 287.7 | 22.8 | 10.4 | 0.0 | 16.9 |

From Table 5 above, it can be seen that all samples have a higher concentration of components exhibiting maximum absorption at 280 nm followed by components absorbing most at 320 nm and then 370 nm. This indicates that the samples are dominated by phenolic acids.

### Conclusion

Eight phenolic compounds were identified in the samples analysed by HPLC-DAD. The high phenolic content of these samples indicates that they would be appropriate for use as preservatives for food or pharmaceuticals.

### Example 3

This example compares the antioxidant capacity of a molasses extract with a green tea extract (currently used as a food antioxidant).

### Materials and methods

*Sample* Preparation: Samples were ground and approximately 50 mg solubilized in 5 ml of methanol. The samples were vortexed, sonicated for 30 minutes, and centrifuged for 5 minutes (1900 RCF). The supernatant was collected and taken to dryness. Samples were re-solubilized in methanol at 10 mg/ml. The green tea and molasses extracts were water soluble.

*Oxygen Radical Absorbance Capacity (ORAC) assay:* The ORAC assay employed in this study measured the antioxidant scavenging activity in the test sample, against peroxyl radicals induced by 2, 2'-azobis (2-amidinopropane) dihydrochloride (AAPH) at 37 °C.

Fluorescein was used as the fluorescent probe. Hydrophilic ORAC values were determined for the samples.

The extracts/samples were assayed using the ORAC procedure in serial dilution (× 4) with AWA (acetone: water: acetic acid; 70:29.5:0.5), and in quadruplicate, starting with the concentration relevant to the sample, depending on the approximated antioxidant capacity from an initial screen. A green tea extract was included as a positive control, and the extract was prepared as per the sample preparation.

A methanolic green tea extract was included as a positive control, and was solubilized in phosphate buffer (pH 7.4) also.

Trolox, a water soluble analogue of vitamin E, was used as a reference standard. A trolox standard curve was established from trolox standards prepared at 100, 50, 25, and 12.5 µM in AWA.

Briefly, 20 µL samples/standards/control/blank (AWA), 10 µL fluorescein (6.0 × 10⁻⁷ M), and 170 µL AAPH (20 mM) were added to each well. Immediately after loading, the plate was transferred to the plate reader preset to 37°C, and the fluorescence was measured 35 times at one minute intervals. The fluorescence readings were referenced to solvent blank wells. The final ORAC values were calculated using a regression equation between the Trolox concentration and the net area under the fluorescein decay curve, and were expressed as micromole Trolox equivalents (TE) per g of sample.

### Results and discussion

The yield from each product is presented in Table 4.

**Table 4 Yield of extract from each sample**

| **Sample no.** | **Sample mass (mg)** | **Extract mass (mg)** | **Yield (%)** |
|---|---|---|---|
| Molasses extract | 49.8 | 34.3 | 69 |
| Green tea | 48.5 | 16.3 | 34 |

*Antioxidant Capacity:* The antioxidant capacities of the samples, prepared by making methanolic extracts, are presented in Table 5. Molasses extract demonstrated the greatest antioxidant capacity, with an ORAC value of 4395 µmol TE / of sample when an extract was generated or 5020 µmol TE / of sample when dissolved directly in buffer (Table 6). Both values were considerably higher than the corresponding green tea extract.

**Table 5: Antioxidant capacity of molasses extract sample extracted with methanol, compared to a green tea methanol extract**

| **Sample** | **ORAC value µmol TE** / **g of sample** |
|---|---|
| Molasses extract | 4395 ± 229 |
| Green tea | 1793 ± 93.5 |

| | |
|---|---|
| Values are mean ± standard error of the mean. | |

**Table 6: Antioxidant capacity of molasses extract solubilized directly in phosphate buffer (pH 7.4), compared to a green tea methanol extract**

| **Sample** | **ORAC value µmol TE / g of sample** |
|---|---|
| Molasses extract | 5020 ± 375 |
| Green tea | 1467 ± 90 |

| | |
|---|---|
| Values are mean ± standard error of the mean. | |

This example clearly demonstrates that extracts derived from molasses are potent antioxidants with a higher ORAC activity than current commercial natural food antioxidants such as green tea. This indicates that extracts produced according to the invention could be useful as antioxidants and antimicrobials in foods and pharmaceuticals.

### Example 4

This example investigates the distribution of polyphenolic compounds through molasses fractions versus colour in order to produce a low colour food antioxidant.

### Method

Reagents:
- Folin-Ciocalteu reagent (undiluted)
- 7% sodium carbonate (anhydrous)
- Catechin standard (1mg/ml)

The following standards and samples were prepared for a 96 well plate.
- Add 14µl of diluted sample or standards into the assay tubes.
- Add 182µl of ultrapure water and mix.
- Add 14µl of Folin-Ciocalteu reagent.
- Mix and stand for 5 mins.
- Add 140µl of 7% sodium carbonate.
- Mix and stand for 90 mins at room temperature.
- Read at λ₇₅₀ nm. Aim to read the tubes at 90±5 min after adding sodium carbonate.

Standard curve for phenolics assay: Frozen standard of 1 mg/ml catechins. Standard dilutions were prepared as follows:

**Table 7: Catechin standard curve sample dilutions.**

| **Catechin (µL of 1 mg/ml)** | **Water (µl)** | **Catechin Concentration (µg/ml)** |
|---|---|---|
| 0 | 1000 | 0 |
| 50 | 950 | 50 |
| 100 | 900 | 100 |
| 150 | 850 | 150 |
| 200 | 800 | 200 |
| 250 | 750 | 250 |

Samples: Samples chosen for the experiment were the fractions 3-26 from the following gel permeation.
- Molasses was diluted in a 1:4 ratio in 0.1 M sodium chloride solution.
- 0.35 ml samples were injected and run through a P2 gel at 0.35 ml/min, collecting 3 ml fractions.
- Fractions were stored at 4°C for 36 hours prior to analysis.
- Fractions were allowed to reach room temperature and mixed via vortex before being sampled.
- Samples were tested on a 96-well plate that included the catechin standards.
- Each mixing step was achieved using the mixing mechanism present in the microplate reader.
- The plate was read using a BioRad Model 680XR microplate reader, using the endpoint function in single reading mode with a 750 nm filter.

### Results and Discussion

Two "raw data" reports were compared to ascertain if there was a significant change in the samples due to the extra time before the second reading. The absorbance readings were quite close and so the result was deemed to be accurate.

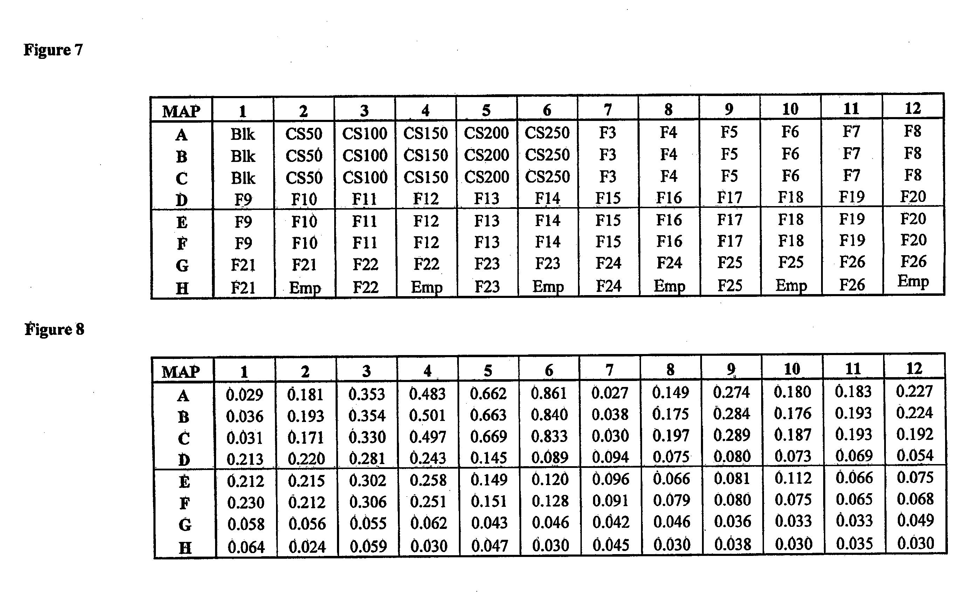

The results are illustrated in Figures 7 to 16 and Tables 8 to 12.

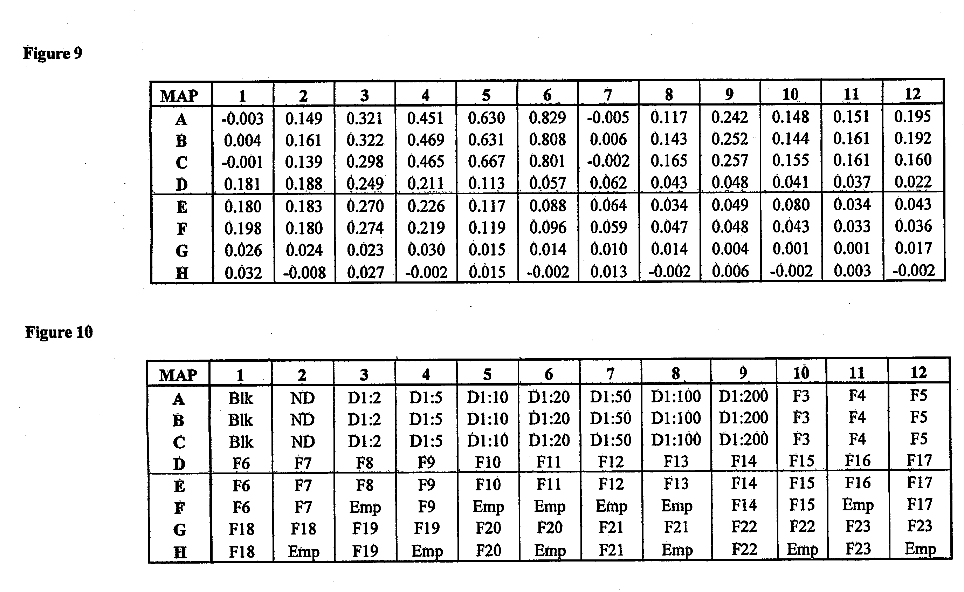

The plate map and absorbance readings are in Figures 7 to 9. In Figure 7, blanks (Blk), catechin standards (CS...) and fraction samples (F) are all in triplicate with empty wells denoted by "Emp". Figure 9 shows the corrected absorbance values where the blanks have been realised and subtracted from the standards and samples.

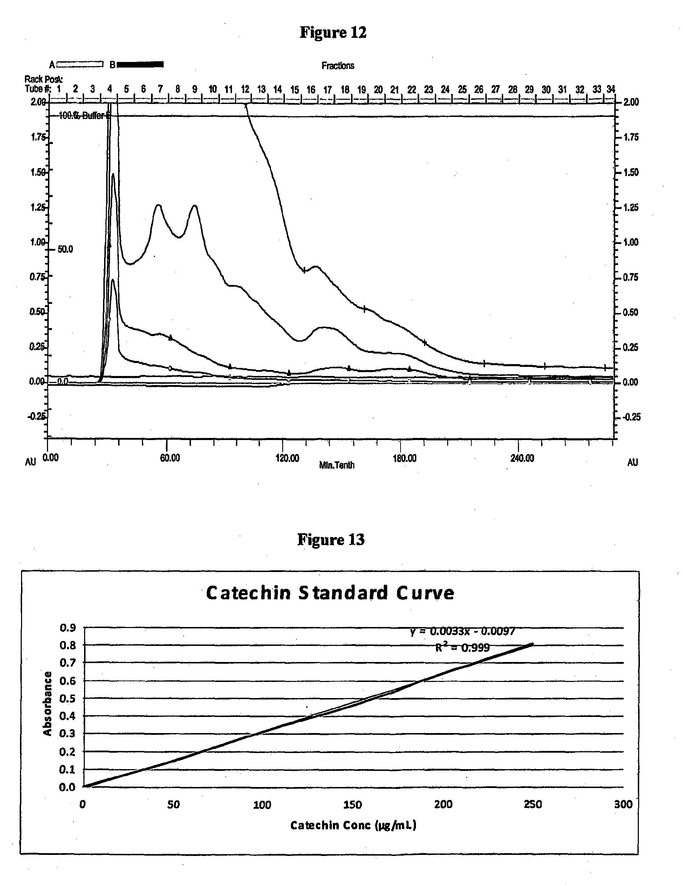

The catechin standard curve obtained is very linear given the R² value of 0.999 (see Table 8 and Figure 13).

**Table 8: Data for the catechin standard curve.**

| **Conc (µg/ml)** | **Abs 1** | **Abs 2** | **Abs 3** | **Abs Ave** | **SD** | **%CV** |
|---|---|---|---|---|---|---|
| **0** | -0.003 | 0.004 | -0.001 | 0.0000 | 0.0036 | #DIV/0! |
| **50** | 0.149 | 0.161 | 0.139 | 0.1497 | 0.0110 | 15% |
| **100** | 0.321 | 0.322 | 0.298 | 0.3137 | 0.0136 | 9% |
| **150** | 0.451 | 0.469 | 0.465 | 0.4617 | 0.0095 | 4% |
| **200** | 0.630 | 0.631 | 0.667 | 0.6427 | 0.0211 | 7% |
| **250** | 0.829 | 0.808 | 0.801 | 0.8127 | 0.0146 | 4% |

**Table 9: Raw data for the fraction samples, with their catechin equivalence in the right column. (see Figure 8)**

| **Fraction** | **Abs 1** | **Abs 2** | **Abs 3** | **Abs Ave** | **SD** | **2SD** | **%CV** | **Cat Equiv** |
|---|---|---|---|---|---|---|---|---|
| **3** | -0.005 | 0.006 | -0.002 | -0.0003 | 0.0057 | 0.0114 | -3412% | 2.8384 |
| **4** | 0.117 | 0.143 | 0.165 | 0.1417 | 0.0240 | 0.0481 | 34% | 45.8687 |
| **5** | 0.242 | 0.252 | 0.257 | 0.2503 | 0.0076 | 0.0153 | 6% | 78.7980 |
| **6** | 0.148 | 0.144 | 0.155 | 0.1490 | 0.0056 | 0.0111 | 7% | 48.0909 |
| **7** | 0.151 | 0.161 | 0.161 | 0.1577 | 0.0058 | 0.0115 | 7% | 50.7172 |
| **8** | 0.195 | 0.192 | 0.160 | 0.1823 | 0.0194 | 0.0388 | 21% | 58.1919 |
| **9** | 0.181 | 0.180 | 0.198 | 0.1863 | 0.0101 | 0.0202 | 11% | 59.4040 |
| **10** | 0.188 | 0.183 | 0.180 | 0.1837 | 0.0040 | 0.0081 | 4% | 58.5960 |
| **11** | 0.249 | 0.270 | 0.274 | 0.2643 | 0.0134 | 0.0269 | 10% | 83.0404 |
| **12** | 0.211 | 0.226 | 0.219 | 0.2187 | 0.0075 | 0.0150 | 7% | 69.2020 |
| **13** | 0.113 | 0.117 | 0.119 | 0.1163 | 0.0031 | 0.0061 | 5% | 38.1919 |
| **14** | 0.057 | 0.088 | 0.096 | 0.0920 | 0.0057 | 0.0113 | 12% | 30.8182 |
| **15** | 0.062 | 0.064 | 0.059 | 0.0617 | 0.0025 | 0.0050 | 8% | 21.6263 |
| **16** | 0.043 | 0.034 | 0.047 | 0.0413 | 0.0067 | 0.0133 | 32% | 15.4646 |
| **17** | 0.048 | 0.049 | 0.048 | 0.0483 | 0.0006 | 0.0012 | 2% | 17.5859 |
| **18** | 0.041 | 0.080 | 0.043 | 0.0420 | 0.0014 | 0.0028 | 7% | 15.6667 |
| **19** | 0.037 | 0.034 | 0.033 | 0.0347 | 0.0021 | 0.0042 | 12% | 13.4444 |
| **20** | 0.022 | 0.043 | 0.036 | 0.0337 | 0.0107 | 0.0214 | 64% | 13.1414 |
| **21** | 0.026 | 0.032 | 0.024 | 0.0273 | 0.0042 | 0.0083 | 30% | 11.2222 |
| **22** | 0.023 | 0.027 | 0.030 | 0.0267 | 0.0035 | 0.0070 | 26% | 11.0202 |
| **23** | 0.015 | 0.015 | 0.014 | 0.0147 | 0.0006 | 0.0012 | 8% | 7.3838 |
| **24** | 0.010 | 0.013 | 0.014 | 0.0123 | 0.0021 | 0.0042 | 34% | 6.6768 |
| **25** | 0.004 | 0.006 | 0.001 | 0.0037 | 0.0025 | 0.0050 | 137% | 4.0505 |
| **26** | 0.001 | 0.003 | 0.017 | 0.0070 | 0.0087 | 0.0174 | 249% | 5.0606 |

Plotting the catechin equivalence of the fractions with ±2SD as error requires that each triplicate absorbance be changed into catechin equivalence and the SD of those numbers used to represent error.

The values in Table 9 were recalculated for this purpose, also taking F14 rep1 and F18 rep2 values out of the calculations as they were noted errors.

**Table 10: Data for the fraction samples, in catechin equivalence (plate 1).**

| **Fraction** | **CE1** | **CE2** | **CE3** | **CE Ave** | **SD** | **2SD** | **%CV** |
|---|---|---|---|---|---|---|---|
| **3** | 1.424 | 4.758 | 2.333 | 2.8384 | 1.7231 | 3.4462 | 121% |
| **4** | 38.394 | 46.273 | 52.939 | 45.8687 | 7.2811 | 14.5623 | 32% |
| **5** | 76.273 | 79.303 | 80.818 | 78.7980 | 2.3144 | 4.6289 | 6% |
| **6** | 47.788 | 46.576 | 49.909 | 48.0909 | 1.6872 | 3.3744 | 7% |
| **7** | 48.697 | 51.727 | 51.727 | 50.7172 | 1.7495 | 3.4991 | 7% |
| **8** | 62.030 | 61.121 | 51.424 | 58.1919 | 5.8786 | 11.7572 | 20% |
| **9** | 57.788 | 57.485 | 62.939 | 59.4040 | 3.0655 | 6.1309 | 10% |
| **10** | 59.909 | 58.394 | 57.485 | 58.5960 | 1.2247 | 2.4494 | 4% |
| **11** | 78.394 | 84.758 | 85.970 | 83.0404 | 4.0693 | 8.1387 | 10% |
| **12** | 66.879 | 71.424 | 69.303 | 69.2020 | 2.2744 | 4.5488 | 7% |
| **13** | 37.182 | 38.394 | 39.000 | 38.1919 | 0.9258 | 1.8515 | 5% |
| **14** | 20.212 | 29.606 | 32.030 | 30.8182 | 1.7142 | 3.4284 | 11% |
| **15** | 21.727 | 22.333 | 20.818 | 21.6263 | 0.7626 | 1.5252 | 7% |
| **16** | 15.970 | 13.242 | 17.182 | 15.4646 | 2.0177 | 4.0354 | 26% |
| **17** | 17.485 | 17.788 | 17.485 | 17.5859 | 0.1750 | 0.3499 | 2% |
| **18** | 15.364 | 27.182 | 15.970 | 15.6667 | 0.4285 | 0.8571 | 5% |
| **19** | 14.152 | 13.242 | 12.939 | 13.4444 | 0.6308 | 1.2616 | 9% |
| **20** | 9.606 | 15.970 | 13.848 | 13.1414 | 3.2402 | 6.4804 | 49% |
| **21** | 10.818 | 12.636 | 10.212 | 11.2222 | 1.2616 | 2.5232 | 22% |
| **22** | 9.909 | 11.121 | 12.030 | 11.0202 | 1.0642 | 2.1284 | 19% |
| **23** | 7.485 | 7.485 | 7.182 | 7.3838 | 0.1750 | 0.3499 | 5% |
| **24** | 5.970 | 6.879 | 7.182 | 6.6768 | 0.6308 | 1.2616 | 19% |
| **25** | 4.152 | 4.758 | 3.242 | 4.0505 | 0.7626 | 1.5252 | 38% |
| **26** | 3.242 | 3.848 | 8.091 | 5.0606 | 2.6418 | 5.2835 | 104% |

The plates were reproduced twice again to ensure that this is a fair representation of the results.

**Table 11: Data for the fraction samples in catechin equivalence (plate 2)**

| **Fraction** | **CE1** | **CE2** | **CE3** | **CE Ave** | **SD** | **2SD** | **%CV** |
|---|---|---|---|---|---|---|---|
| **3** | -0.118 | 2.235 | 0.176 | 0.7647 | 1.2820 | 2.5641 | 335% |
| **4** | 36.647 | 44.000 | 44.294 | 41.6471 | 4.3326 | 8.6652 | 21% |
| **5** | 76.941 | 79.000 | 75.765 | 77.2353 | 1.6376 | 3.2752 | 4% |
| **6** | 44.882 | 50.765 | 50.765 | 48.8039 | 3.3962 | 6.7924 | 14% |
| **7** | 43.706 | 47.235 | 50.765 | 47.2353 | 3.5294 | 7.0588 | 15% |
| **8** | 51.059 | 52.529 | 55.765 | 53.1176 | 2.4075 | 4.8149 | 9% |
| **9** | 55.765 | 59.000 | 57.529 | 57.4314 | 1.6199 | 3.2397 | 6% |
| **10** | 53.412 | 57.235 | 61.353 | 57.3333 | 3.9715 | 7.9430 | 14% |
| **11** | 76.059 | 68.706 | 74.294 | 73.0196 | 3.8386 | 7.6772 | 11% |
| **12** | 64.882 | 40.471 | 67.529 | 66.2059 | 1.8718 | 3.7435 | 6% |
| **13** | 35.765 | 35.176 | 36.647 | 35.8627 | 0.7402 | 1.4804 | 4% |
| **14** | 26.059 | 29.882 | 28.412 | 28.1176 | 1.9287 | 3.8573 | 14% |
| **15** | 20.765 | 21.059 | 21.941 | 21.2549 | 0.6123 | 1.2245 | 6% |
| **16** | 14.000 | 15.471 | 15.471 | 14.9804 | 0.8490 | 1.6981 | 11% |
| **17** | 15.471 | 17.529 | 19.000 | 17.3333 | 1.7729 | 3.5457 | 20% |
| **18** | 14.588 | 14.588 | 15.765 | 14.9804 | 0.6792 | 1.3585 | 9% |
| **19** | 11.353 | 10.471 | 10.471 | 10.7647 | 0.5094 | 1.0189 | 9% |
| **20** | 7.529 | 11.941 | 13.412 | 10.9608 | 3.0613 | 6.1225 | 56% |
| **21** | 11.059 | 10.176 | 11.059 | 10.7647 | 0.5094 | 1.0189 | 9% |
| **22** | 8.118 | 7.529 | 9.294 | 8.3137 | 0.8985 | 1.7971 | 22% |
| **23** | 6.353 | 6.647 | 6.941 | 6.6471 | 0.2941 | 0.5882 | 9% |
| **24** | 4.588 | 4.294 | 4.588 | 4.4902 | 0.1698 | 0.3396 | 8% |
| **25** | 3.412 | 2.824 | 2.529 | 2.9216 | 0.4493 | 0.8985 | 31% |
| **26** | 1.353 | 1.353 | 5.765 | 2.8235 | 2.5471 | 5.0943 | 180% |

**Table 12: Data for the fraction samples in catechin equivalence (plate 3)**

| **Fraction** | **CE 1** | **CE2** | **CE 3** | **CE Ave** | **SD** | **2SD** | **%CV** |
|---|---|---|---|---|---|---|---|
| **3** | -3.800 | 4.200 | -0.371 | 1.9143 | 3.2325 | 6.4650 | 338% |
| **4** | 36.771 | 44.486 | 42.771 | 41.3429 | 4.0507 | 8.1014 | 20% |
| **5** | 73.343 | 65.057 | 75.057 | 71.1524 | 5.3478 | 10.6955 | 15% |
| **6** | 46.200 | 50.771 | 48.200 | 48.3905 | 2.2917 | 4.5833 | 9% |
| **7** | 49.343 | 51.057 | 49.343 | 49.9143 | 0.9897 | 1.9795 | 4% |
| **8** | 52.486 | 55.629 | 54.486 | 54.2000 | 1.5908 | 3.1816 | 6% |
| **9** | 54.486 | 57.057 | 51.343 | 54.2952 | 2.8619 | 5.7238 | 11% |
| **10** | 58.200 | 54.200 | 65.343 | 59.2476 | 5.6448 | 11.2896 | 19% |
| **11** | 81.057 | 78.200 | 78.486 | 79.2476 | 1.5736 | 3.1472 | 4% |
| **12** | 63.057 | 62.200 | 65.057 | 63.4381 | 1.4662 | 2.9323 | 5% |
| **13** | 35.914 | 36.486 | 37.057 | 36.4857 | 0.5714 | 1.1429 | 3% |
| **14** | 25.057 | 27.914 | 27.343 | 26.7714 | 1.5119 | 3.0237 | 11% |
| **15** | 19.629 | 20.200 | 20.486 | 20.1048 | 0.4364 | 0.8729 | 4% |
| **16** | 13.914 | 13.629 | 14.200 | 13.9143 | 0.2857 | 0.5714 | 4% |
| **17** | 15.914 | 16.771 | 15.343 | 16.0095 | 0.7190 | 1.4381 | 9% |
| **18** | 15.629 | 15.057 | 14.200 | 14.9619 | 0.7190 | 1.4381 | 10% |
| **19** | 11.343 | 11.057 | 12.771 | 11.7238 | 0.9184 | 1.8369 | 16% |
| **20** | 8.200 | 11.343 | 11.343 | 10.2952 | 1.8145 | 3.6291 | 35% |
| **21** | 10.486 | 8.771 | 10.771 | 10.0095 | 1.0817 | 2.1634 | 22% |
| **22** | 8.486 | 9.914 | 9.629 | 9.3429 | 0.7559 | 1.5119 | 16% |
| **23** | 5.914 | 5.629 | 6.486 | 6.0095 | 0.4364 | 0.8729 | 15% |
| **24** | 3.629 | 3.914 | 3.914 | 3.8190 | 0.1650 | 0.3299 | 9% |
| **25** | 2.200 | 2.771 | 1.914 | 2.2952 | 0.4364 | 0.8729 | 38% |
| **26** | 0.771 | 3.343 | 4.771 | 2.9619 | 2.0270 | 4.0541 | 137% |

The samples were tested in triplicate, each plate having its own catechin standard reference curve. Figure 9 plots the polyphenolic results of each fraction from each plate and confirms the accuracy of the assay.

There are no significant outlying results, with respect to the ±2SD error of each of the fractions. This indicates that the assay is relatively accurate, even though the absorbance readings of each fraction triplicate is not entirely precise, as seen by the %CV values.

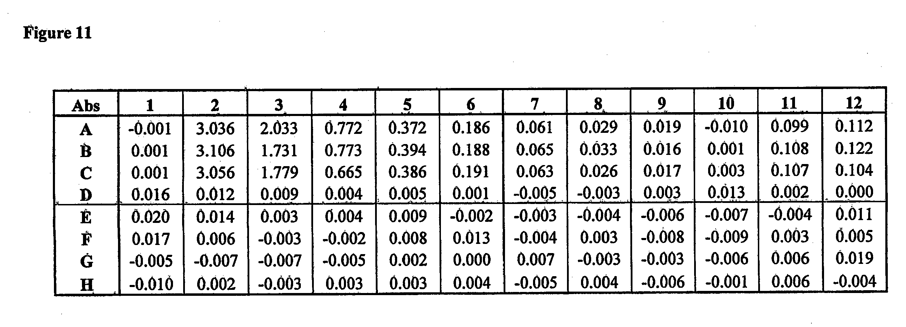

The absorbance of the fractions were analysed using a microplate reader at 750 nm on 0.35 ml samples. Figure 10 shows the map of 96 well plate of Molasses feedstock dilutions and fractions 3-23. (In Figure 10: ND- no dilution, D1:2 - 1 in 2 dilution, F - fraction number, Emp - empty well.) Figure 11 shows the absorbance values at 750 nm for the map in Figure 10.

Figure 12 shows a chromatogram of run described above. Orange, purple, green and blue traces show λ405, 350, 280 and 214 nm respectively. Collected fractions are shown on top axis.

### Legend for Figure 12

| **Line style** | **Wavelength (nm)** |
|---|---|
| 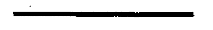 | 214 |
| 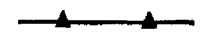 | 280 |
| 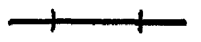 | 350 |
| 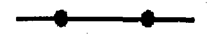 | 405 |

At this wavelength (750 nm), fractions 8 onwards have absorbance values below 0.010, which is considered to be 'low colour'.

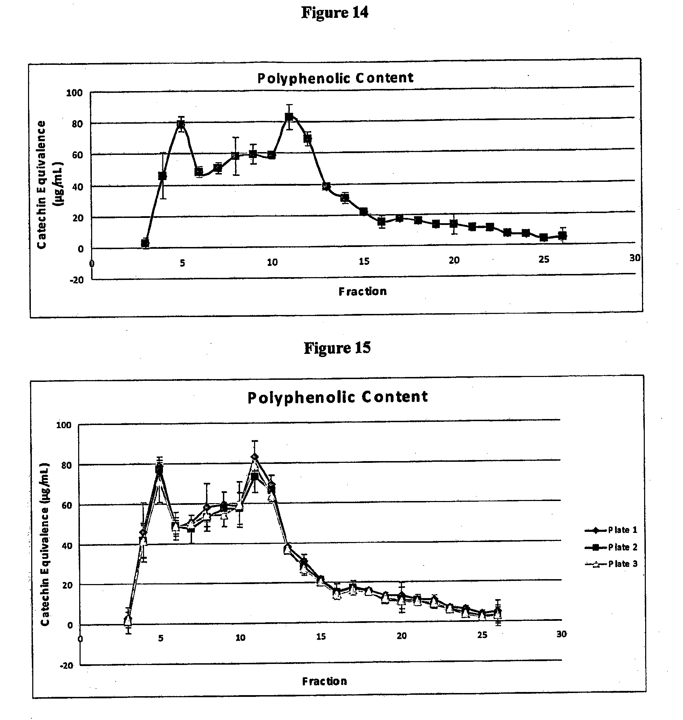

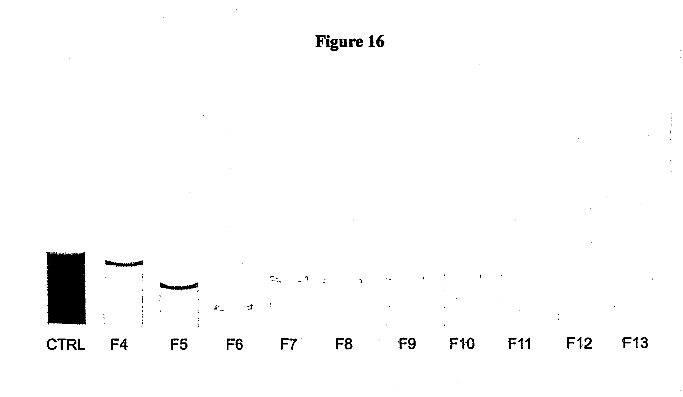

In Figure 15, it can be seen that fraction 12 gives a fairly high polyphenolic content with respect to catechin equivalence and that this fraction also coincides with a shoulder peak on the λ280 nm trace in Figure 12. The λ405 nm trace for fraction 12 in Figure 12 is quite low, indicating low colour. This colour difference of the fractions can be seen in Figure 16.

### Conclusion

Fractions considered to be 'high antioxidant' contained a polyphenolic content of 50µg/ml catechin equivalence or higher under these assay conditions.

Fractions considered to be 'low colour' had an absorbance reading of 0.010 or lower under these assay conditions.

This 750 nm wavelength was used for the polyphenolic reaction. The absorbance of the actual colour of the fractions would have been better analysed at 415 nm as the colour absorbs more energy at this wavelength, making it more sensitive to differences between fractions. However, the measurements at 750 nm provide a useful indication of the colour measurement.

This experiment demonstrates that a range of low colour high antioxidant fractions can be isolated from molasses. Figure 16 shows some of the low colour fractions compared with the control (molasses). None of the low colour samples have a noticeable smell. The low colour high antioxidant fractions can be used in a variety of applications without interfering with organoleptic aspects of finished foods due to their low colour.

### Example 5

A toothpaste according to the invention for use in the method of treatment or prevention of dental caries and oral hygiene was prepared as follows:

| | **Ingredients** | **%w/w** |
|---|---|---|
| A) | Sorbitol USP | 15.0 |
| | Casein Phosphopeptide (CPP) | 7.5 |
| | Low colour high antioxidant molasses extract | 0.5 |
| B) | Glycerin USP 96% | 10.0 |
| | Triclosan | 0.3 |
| | Na-Saccharin USP 40/60 Mesh | 0.2 |
| | Veegum D-Granular | 2.0 |
| | Peppermint Oil | 1.1 |
| | Stepanol WA/100 (Na-Lauryl Sulfate) | 2.2 |
| C) | Veegum HF-6% (Ag/Al Silicate) | 16.64 |
| | Blue #1 FD+C (0.6%) | 0.06 |
| D) | Na-CMC 7 H 5% | 45.0 |

The low colour high antioxidant molasses extract was supplied as a slightly brown watersoluble free flowing powder. The components of A were combined together and then all items of B were added to A and mixed at 70°C until uniform. C was then added and mixed until uniform. Finally, D was added slowly with mixing until uniform. Citric acid q.s. to pH 5.9 to 6.3.

### Example 6

The toothpaste of Example 5 above containing the addition of 0.3% sodium monofluorophosphate for children

### Example 7

The toothpaste of Example 5 above containing the addition of a tooth whitening compound.

### Example 8

This composition provides a mouthwash.

| **Ingredients** | **%w/w** |
|---|---|
| Calcium phosphate | 2.0 |
| Low colour high antioxidant molasses extract | 0.5 |
| Poloxamer | 1.0 |
| Flavour | q.s. |
| Water/Ethanol | q.s. ad 100% |

### Example 9

This composition provides a chewing gum.

| **Ingredients** | **%w/w** |
|---|---|
| Cane Sugar or low GI sugar citied in WO 2005/117608 | 2.0 |
| Low colour high antioxidant molasses extract | 1.5 |
| Gum Base | q.s. |
| Wheat glucose syrup | 0.5 |
| Food acid (296) | 1.0 |
| Humectant (422) | 2.0 |
| Flavour | q.s. |
| Emulsifier (322 from Soy) | 0.5 |
| Colours (100,133) | 0.0002 |
| Antioxidant (BHT) | 0.1 |

### Example 10

This composition provides a soft gelatine confectionary.

| **Ingredients** | **%w/w** |
|---|---|
| Wheat Glucose Syrup | 36% |
| Cane Sugar or low GI sugar citied in WO 2005/117608 | 32% |
| Wheat or Corn Starch | 23% |
| Gelatine | 6% |
| Citric acid | 0.95% |
| Fruit juice concentrate | qs |
| Natural flavours | qs |
| Natural colors | qs |
| Low colour high antioxidant molasses extract | 2.0% |

### Example 11

This composition provides a flavoured water beverage.

| **Ingredients** | **Weight (g)** |
|---|---|
| Sugar | 8.50 |
| Low colour high antioxidant molasses extract | 2.00 |
| Citric acid 50% w/w | 2.00 |
| Flavour | 0.30 |
| Sodium benzoate | 0.10 |
| Water | to 1000 ml |

### Preparation:

- Dissolve sodium benzoate by stirring in 50 mls of water
- Add citric acid solution and low colour high antioxidant molasses extract
- Stir until low colour high antioxidant molasses extract has dissolved
- Add sugar and stir until dissolved then add flavour and stir until blended.
- Add water to one litre

### Example 12

This composition provides a fruit puree snack.

| **Ingredients** | **%w/w** |
|---|---|
| Apple Puree | 79.00 |
| Raspberry Puree | 12.00 |
| Apple juice concentrate | 7.6 |
| Low colour high antioxidant molasses extract | 1.2 |
| Raspberry flavour | 0.2 |
| Total | 100 |

### Preparation:

- Blend purees and juice concentrate together
- Add molasses extract and flavour. Mix well until blended

### Example 13

This composition provides an oil with added molasses antioxidant.

| **Ingredients** | **%w/w** |
|---|---|
| Sunflower oil | 98.8 |
| Low colour high antioxidant molasses extract | 1.2 |
| Total | 100 |

### Example 14

This composition provides a cereal bar.

| **Ingredients** | **%w/w** |
|---|---|
| Skim-milk powder | 2.00 |
| Low colour high antioxidant molasses extract | 0.50 |
| Rice crisps | 20.00 |
| Wheat crisps | 10.00 |
| Apples, dried and diced | 6.00 |
| Sultanas | 3.00 |
| Almonds, roasted and diced | 4.00 |
| Glucose syrup DE38, 43°Be | 11.00 |
| Invert sugar syrup (74-76%) | 5.25 |
| Sorbitol syrup | 20.0 |
| Palm kernel fat | 2.00 |
| Lecithin | 0.15 |
| Sugar | 12.00 |
| Water | 4.00 |
| Salt | 0.10 |
| Total | 100 |

### Preparation:

- Mix low colour high antioxidant molasses extract with skim-milk powder and place in a Hobart mixer
- Add rice and wheat crisps and mix gently with powder ingredients. Then add the fruit ingredients and mix
- Mix glucose syrup, invert syrup and sorbitol syrup and heat up to 113°C. Then cool down in a cold water bath in order to stop the cooking process
- Melt palm kernel fat and Lecithin in a water bath at 75°C
- Add fat mixture to the syrup combination
- Mix sugar, water and salt and heat up to 110°C
- Add fat to the sugar solution
- Add liquid mass to dry ingredients in the Kenwood-type mixer and mix well
- Put the mass on a marble plate and roll to the desired thickness. Let the mass cool down at room temperature
- Cut into pieces of one serving size and pack

### Example 15

To determine the antimicrobial activity of a molasses extract polyphenol powder against food spoilage and oral hygiene microorganisms.

### Methods

*Molasses extract polyphenol powder:* Polyphenol powder from molasses (5g) was provided by Horizon Science Pty Ltd as described in international patent application no WO 2005/117608.

*Bacterial strains and growth conditions*: Test organisms used in the assay were *Staphylococcus aureus* strain 6571 (NCTC-National Collection of Type Cultures, Health Protection Agency Centre for Infection, London, UK), *Streptococcus mutans* ACM 969 and *Proteus vulgaris* ACM 4730, were supplied by the Australian Collection of Microorganisms. (ACM), University of Queensland. The test organisms were grown for 24 h in Tryptone Soya Yeast Extract Broth (TSYEB) (Oxoid CM 129B, Basingstoke, UK), 30 g/L; yeast extract (Oxoid CM19), 6 g/L. The inoculum was quantified by measuring the optical density (absorbance) at 540 nm and adjusted to 0.5 absorbance with TSYEB.

*Preparation and dilution of bacterial strains*: An inoculum was prepared by serially diluting (1:1) the overnight culture of the test organisms in TSYEB broth 22 times. The dilutions from 11-22 were used on the 96 well microtitre plate. After the dilutions were made the lowest dilution was plated on Plate Count Agar (Oxoid CM0463) for all the test organisms to confirm the bacterial count.

*Preparation of polyphenol powder from molasses*: The polyphenol powder from molasses (0.1g) was dissolved in 10 ml of TSYEB. This solution was diluted in TSYEB starting at a concentration of 1% (w/v) and diluted to 0.0005% (w/v).

*Preparation of antibiotic solutions*: The antibiotics Penicillin G and Oxytetracyclin hydrochloride from Sigma-Aldrich (St. Louis, MO) were used in this study. 0.01 g each of Penicillin G and Oxytetracyclin were dissolved in 20 ml of TSYEB.

The antibiotic solutions of Penicillin G and Oxytetracyclin were diluted in TSYEB (1:1) starting at a concentration of 0.05% (w/v) and diluted to 2.44 x 10⁻⁵% (w/v).

*Microplate assay procedure*: Flat bottom 96 well sterile microtiter plates with lid to prevent cross contamination (Sarstedt, Nümbrecht, Germany) were used for the study. Each 96 well microtiter plate supported one row of wells containing the medium alone (sterility and negative control). Three rows from the first row of wells contained 1:1 dilutions of test organisms (bacterial controls equivalent to 10⁵ cfu/ml in the left most well to 10² cfu/ml in the right most well) and the polyphenol solution from molasses highest to the lowest concentration (1% to 0.0005 % w/v). Each of the combinations of polyphenol solution and bacterial cells were done in triplicate measurements. The next three rows of wells contained the same number of bacterial dilutions but the polyphenol solution was from the lowest to the highest concentration (0.0005% to 1% w/v). Each of the combinations of polyphenol solution and bacterial cells were done in triplicate measurements. The remaining row contained the same number of bacterial dilutions with growth media and polyphenol solution and was called the positive control. Replicates (n=6) of the positive control with no polyphenol solution was run on a separate plate for test organisms. Each 300 µl well contained 50 µl of inoculum, 150 µL of polyphenol solution. Each negative or sterility control well contained 200 µl of TSYEB broth. Each positive control well contained 50 µl of inoculum, 150 µl of TSYEB broth.

Penicillin G and Oxytetracyclin hydrochloride were used as reference standards to determine the sensitivity of test organisms and to compare the antimicrobial activity of the polyphenol solution with that of the antibiotics.

Optical density (OD) was determined in a spectrophotometer Sunrise-Basic Tecan (Grödig, Austria) at 540 nm. OD was determined prior to incubation and represents the spectrophotometric reading at time zero (*To*)*.* Plates were placed in an incubator at 37°C and incubated for 22 h. *Streptococcus mutans* was incubated at 37°C for 22 and 44 h. The solutions in the plates were mixed using a multichannel pipette to prevent clumping prior to reading the OD in the spectrophotometer after 22 h (*T₂₂*)*.*

*Analysis of results*: The calculations for Percent Inhibition were based on the study by Casey *et al.* 2004; Patton *et al.* 2006. Percent Inhibition = 1-(OD test well/OD of corresponding positive control well) x 100.
- MIC₀ is the highest concentration of polyphenol solution or antibiotic which results in no inhibition of growth;
- MIC₅₀ is the concentration of polyphenol solution or antibiotic which results in 50% inhibition of growth; and
- MIC₁₀₀ is the lowest concentration polyphenol solution or antibiotic which results in 100% inhibition.

### Results

**Table 13. Results of analysis for antibacterial activity in polyphenol powder from molasses**

| **Test Organism** | **Minimum Inhibitory Concentration (MIC) % w/v (concentration of polyphenol powder)** | | | **Gram Stain** | **Growth condition (respiration)** |
|---|---|---|---|---|---|
| | **MIC₀** | **MIC₅₀** | **MIC₁₀₀** | | |
| *Staphylococcus aureus* | <0.001 | 0.28 | >1 | Positive | Aerobic |
| *Proteus vulgaris* | 0.02 | 1 | >1 | Negative | Aerobic |
| *Streptococcus mutans* | 0.06 | 0.50 | 1 | Positive | Facultative anaerobe |

**Table 14. Results of analysis for antibacterial activity in Penicillin G**

| **Test Organism** | **Minimum Inhibitory Concentration (MIC) % w/v (concentration of penicillin)** | | |
|---|---|---|---|
| | **MIC₀** | **MIC₅₀** | **MIC₁₀₀** |
| *Staphylococcus aureus* | <2.44 x 10⁻⁵ | 4.88 x 10⁻⁵ | 9.77 x 10⁻⁵ |
| *Proteus vulgaris* | 4.88 x 10⁻⁵ | 1.95 x 10⁻⁴ | 7.81 x 10⁻⁴ |
| *Streptococcus mutans* | - | - | <2.44 x 10⁻⁵ |

**Table 15. Results of analysis for antibacterial activity in Oxytetracylin**

| **Test Organism** | **Minimum Inhibitory Concentration (MIC) % w/v (concentration of oxytetracyclin)** | | |
|---|---|---|---|
| | **MIC₀** | **MIC₅₀** | **MIC₁₀₀** |
| *Staphylococcus aureus* | - | - | <2.44 x 10⁻⁵ |
| *Proteus vulgaris* | 2.44 x 10⁻⁵ | 3.91 x 16⁻⁴ | 7.88 x 10⁻⁴ |
| *Streptococcus mutans* | - | - | <2.44 x 10⁻⁵ |

### Discussion

Preliminary screening of the polyphenol solution for antibacterial activity was done against *Staphylococcus aureus.* Through this screening the highest concentration of polyphenol solution that could be used was determined. A concentration greater than 1 % (w/v) of polyphenol powder gave a reading for optical density higher than 1 which is beyond the range recommended for spectrophotometric analysis. The polyphenol solution showed inhibition against *Staphylococcus aureus* at MIC₀ < 0.001%, MIC₅₀ at 0.28% and MIC₁₀₀ >1% (w/v), refer to Table 13.

*Proteus vulgaris* is a food spoilage organism which indicated inhibition against the polyphenol solution revealing a MIC₀ 0.02%, MIC₅₀ at 1% and MIC₁₀₀ >1% (w/v), refer to Table 13. *Streptococcus mutans* did not grow in 22 hours so the period of growth was extended to 44 h. *Streptococcus mutans* revealed complete inhibition at 1%, MIC₅₀ at 0.5% and MIC₀ at 0.06% (w/v), refer to Table 13.

The antibiotics used as reference standards indicate a much lower concentrations for complete inhibition, however different test organisms required different concentrations. *Staphylococcus aureus* and *Streptococcus mutans* were completely inhibited at the lowest concentrations of oxytetracyclin (2.44 x 10⁻⁵% w/v), refer to Table 15. These two Gram positive organisms were also inhibited by the molasses extract polyphenol powder.

### Conclusion

A food spoilage organism *Proteus vulgaris* was inhibited by the molasses extract polyphenol powder.

Streptococcus *mutans* and *Staphylococcus aureus* are both pathogens and their growth was inhibited by the molasses extract polyphenol powder.

The 1% level required for inhibition by the molasses extract polyphenolic powder could be considered very weak in comparison with other plant extracts with useful efficacy in the prevention of microbial growth. The batch used in this study was not recently prepared and a fresh batch of the molasses extract polyphenol powder may yield better results as polyphenols tend to oxidize rapidly without appropriate storage.

Whilst the example uses a high colour molasses extract, it is expected that the relevant polyphenols will be present in the low colour high antioxidant extracts used in the methods according to the invention. From these results, it can be inferred that the low colour high antioxidant extracts derived from sugar cane will be useful in inhibiting the formation of dental caries and improving oral hygiene.

### References

Casey JT, O'Cleirigh C, Walsh PK, O'Shea DG, Development of a robust microtiter plate-based assay method for assessment of bioactivity, Journal of Microbiological Methods 58 (2004) 327-334.

Patton T, Barrett J, Brennan J, Moran N, Use of a spectrophotometric bioassay for determination of microbial sensitivity to manuka honey, *Journal of Microbiological Methods* **64**

The word 'comprising' and forms of the word 'comprising' as used in this description and in the claims does not limit the invention claimed to exclude any variants or additions.

## Claims

1. A preservative comprising a low colour polyphenol enriched molasses extract derived from sugar cane which has a high antioxidant activity wherein the extract has a polyphenolic content of at least about 50 µg/ml catechin equivalents and the extract is low in colour compared to molasses.

2. The preservative according to claim 1 wherein the extract has an absorbance value of less than or equal to about 0.010 when measured at 750 nm.

3. A method of preserving food, cosmetics and pharmaceuticals comprising the step of adding to the food, cosmetic or pharmaceutical an effective preserving amount of a preservative according to claim 1 or 2.

4. The method according to claim 3 wherein the effective preserving amount is in the range from 0.0001 to 5.0 % by weight of the total composition.

5. The method according to claim 4 wherein the effective preserving amount is in the range from 0.01 to 2.5 % by weight of the total composition.

6. A preservative for use in improving oral hygiene and/or inhibiting, treating and/or preventing the formation of dental caries wherein a preservative according to claim 1 or 2 is added in a therapeutically effective amount to an oral hygiene product.

7. The preservative for use according to claim 6 wherein the therapeutically effective amount is in the range from 0.0001 to 5.0 % by weight of the total composition.

8. The preservative for use according to claim 7 wherein the therapeutically effective amount is in the range from 0.01 to 2.5 % by weight of the total composition.

9. An oral hygiene product containing a preservative according to claim 1 or 2.

10. Use of a preservative according to claim 1 or 2 in the preparation of a medicament as an antioxidant and/or antimicrobial in food, cosmetics or pharmaceuticals.

11. Use of a therapeutically effective amount of a preservative according to claim 1 or 2 in the preparation of an oral hygiene product for improving oral hygiene and/or inhibiting, treating and/or preventing the formation of dental caries.

## Patentansprüche

1. Konservierungsmittel, umfassend ein polyphenolangereichertes Melassenextrakt geringer Farbstärke, das von Zuckerrohr abgeleitet ist, das eine hohe antioxidative Aktivität aufweist, wobei das Extrakt einen Polyphenolgehalt von zumindest ungefähr 50 µg/ml Catechinäquivalente aufweist und das Extrakt im Vergleich zu Melassen eine geringe Farbstärke aufweist.

2. Konservierungsmittel nach Anspruch 1, wobei das Extrakt einen Absorbanzwert von kleiner gleich ungefähr 0,010 bei einer Messung bei 750 nm aufweist.

3. Verfahren zum Konservieren von Lebensmitteln, Kosmetika und Pharmazeutika, umfassend den Schritt des Hinzufügens einer wirksamen konservierenden Menge eines Konservierungsmittels nach Anspruch 1 oder 2 zum Lebensmittel, Kosmetikum oder Pharmazeutikum.

4. Verfahren nach Anspruch 3, wobei die wirksame konservierende Menge im Bereich von 0,0001 bis 5,0 Gew.-% der Gesamtzusammensetzung liegt.

5. Verfahren nach Anspruch 4, wobei die wirksame konservierende Menge im Bereich von 0,01 bis 2,5 Gew.-% der Gesamtzusammensetzung liegt.

6. Konservierungsmittel zur Verwendung bei der Verbesserung der Mundhygiene und/oder bei der Hemmung, Behandlung und/oder Prävention der Bildung von Zahnkaries, wobei ein Konservierungsmittel nach Anspruch 1 oder 2 in einer therapeutisch wirksamen Menge zu einem Mundhygieneprodukt hinzugefügt wird.

7. Konservierungsmittel zur Verwendung nach Anspruch 6, wobei die therapeutisch wirksame Menge im Bereich von 0,0001 bis 5,0 Gew.-% der Gesamtzusammensetzung liegt.

8. Konservierungsmittel zur Verwendung nach Anspruch 7, wobei die therapeutisch wirksame Menge im Bereich von 0,01 bis 2,5 Gew.-% der Gesamtzusammensetzung liegt.

9. Mundhygieneprodukt, das ein Konservierungsmittel nach Anspruch 1 oder 2 enthält.

10. Verwendung eines Konservierungsmittels nach Anspruch 1 oder 2 bei der Herstellung eines Medikaments als Antioxidationsmittel und/oder antimikrobielles Mittel in Lebensmitteln, Kosmetika oder Pharmazeutika.

11. Verwendung einer therapeutisch wirksamen Menge eines Konservierungsmittels nach Anspruch 1 oder 2 bei der Herstellung eines Mundhygieneprodukts zur Verbesserung der Mundhygiene und/oder zur Hemmung, Behandlung und/oder Prävention der Bildung von Zahnkaries.

## Revendications

1. Conservateur comprenant un extrait de mélasse enrichi en polyphénol de faible coloration dérivé de la canne à sucre qui possède une forte activité antioxydante, où l'extrait possède une teneur en polyphénol d'au moins environ 50 µg/ml d'équivalents catéchine et l'extrait présente une faible coloration par comparaison à la mélasse.

2. Conservateur selon la revendication 1, dans lequel l'extrait possède une valeur d'absorbance inférieure ou égale à environ 0,010 lorsqu'elle est mesurée à 750 nm.

3. Procédé pour conserver un aliment, des produits cosmétiques et des produits pharmaceutiques, comprenant l'étape qui consiste à ajouter à l'aliment, au produit cosmétique ou au produit pharmaceutique, une quantité conservatrice efficace d'un conservateur selon la revendication 1 ou 2.

4. Procédé selon la revendication 3, dans lequel la quantité conservatrice efficace est dans la plage comprise entre 0,0001 et 5,0 % en poids de la composition totale.

5. Procédé selon la revendication 4, dans lequel la quantité conservatrice efficace est dans la plage comprise entre 0,01 et 2,5 % en poids de la composition totale.

6. Conservateur destiné à être utilisé pour améliorer l'hygiène orale et/ou inhiber, traiter et/ou prévenir la formation de caries dentaires, où un conservateur selon la revendication 1 ou 2 est ajouté en une quantité thérapeutiquement efficace à un produit d'hygiène orale.

7. Conservateur destiné à être utilisé selon la revendication 6, dans lequel la quantité thérapeutiquement efficace est dans la plage comprise entre 0,0001 et 5,0 % en poids de la composition totale.

8. Conservateur destiné à être utilisé selon la revendication 7, dans lequel la quantité thérapeutiquement efficace est dans la plage comprise entre 0,01 et 2,5 % en poids de la composition totale.

9. Produit d'hygiène orale contenant un conservateur selon la revendication 1 ou 2.

10. Utilisation d'un conservateur selon la revendication 1 ou 2 dans la préparation d'un médicament en tant qu'antioxydant et/ou antimicrobien dans un aliment, des produits cosmétiques ou des produits pharmaceutiques.

11. Utilisation d'une quantité thérapeutiquement efficace d'un conservateur selon la revendication 1 ou 2 dans la préparation d'un produit d'hygiène orale pour améliorer l'hygiène orale et/ou inhiber, traiter et/ou prévenir la formation de caries dentaires.
